# EUROPÄISCHE PATENTSCHRIFT

(11) **EP 0 706 518 B1**
(45) Veröffentlichungstag und Bekanntmachung des Hinweises auf die Patenterteilung: **02.01.2002**
(21) Anmeldenummer: 94919665.3
(22) Anmeldetag: 28.06.1994
(51) Int. Cl.: C07D 239/42, A01N 47/36, C07D 239/46, C07D 251/16, C07D 239/48, C07D 239/52, C07D 251/22, C07D 251/52

(54) **ACYLIERTE AMINOPHENYLSULFONYLHARNSTOFFE; DARSTELLUNG UND VERWENDUNG ALS HERBIZIDE UND PFLANZENWACHSTUMSREGULATOREN**
ACYLATED AMINOPHENYL UREA COMPOUNDS, THEIR PREPARATION AND THEIR USE AS HERBICIDES AND PLANT-GROWTH REGULATORS
AMINOPHENYL-UREES ACYLEES, LEUR PREPARATION ET LEUR APPLICATION COMME HERBICIDES ET COMME REGULATEURS DE CROISSANCE VEGETALE

(30) Priorität: 02.07.1993 DE 4322067
(43) Veröffentlichungstag der Anmeldung: 17.04.1996
(73) Patentinhaber: Aventis CropScience GmbH, 65929 Frankfurt am Main (DE)
(72) Erfinder: SCHNABEL, Gerhard, D-63868 Gro wallstadt (DE); WILLMS, Lothar, D-65719 Hofheim (DE); BAUER, Klaus, D-63456 Hanau (DE); BIERINGER, Hermann, D-65817 Eppstein (DE)
(86) Internationale Anmeldenummer: EP9402091
(87) Internationale Veröffentlichungsnummer: WO9501344

(56) Entgegenhaltungen:
- EP-A- 0 116 518
- WO-A-94/10154
- US-A- 4 892 946

## Beschreibung

Die Erfindung betrifft das technische Gebiet der Herbizide und Pflanzenwachstumsregulatoren, insbesondere der Herbizide zur selektiven Bekämpfung von Unkräutern und Ungräsern in Nutzpflanzenkulturen.

Es ist bekannt, daß heterocyclisch substituierte Phenylsulfonylharnstoffe, die am Phenylring eine Amino- bzw. eine funktionalisierte Aminogruppe tragen, herbizide und pflanzenwachstumsregulierende Eigenschaften besitzen (EP-A-1515; US-A-4,892,946; US-A-4,981,509; EP-A-116 518; US-A-4,664,695; US-A-4,632,695).

Außerdem wurden in der deutschen Patentanmeldung P 42 36 902.9 bereits Phenylsulfonylharnstoffe vorgeschlagen, die am Phenylring in 2-Stellung eine Carboxygruppe oder ein Derivat der Carboxygruppe und in 5-Stellung eine N-Alkyl-N-acyl-aminogruppe aufweisen.

Überraschenderweise wurde nun weiterhin gefunden, daß bestimmte heterocyclisch substituierte Phenylsulfonylharnstoffe als Herbizide oder Pflanzenwachstumsregulatoren besonders gut geeignet sind.

Gegenstand der vorliegenden Erfindung sind Verbindungen der Formel (I) und deren Salze, 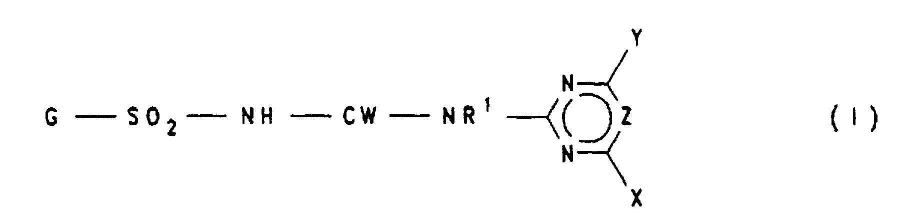 worin
- G: einen substituierten N-Acyl-aminophenylrest aus der Gruppe G¹ und G³, 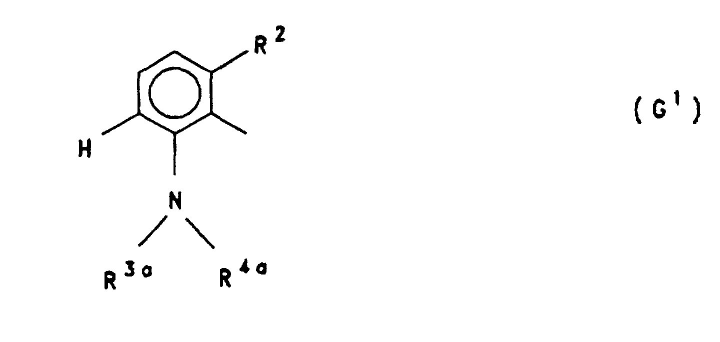 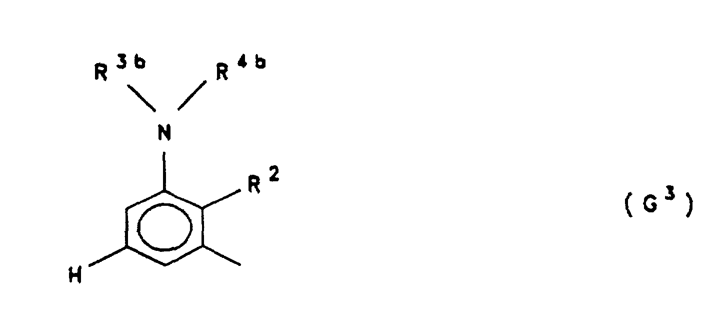
- R¹: H oder C₁-C₃-Alkyl,
- R²: CO-OR⁵, CS-SR⁶, CO-SR⁷, CS-OR⁸, CO-R⁹, CO-NR¹⁰R¹¹, CO-O-N=CR¹²R¹³, C(=NR¹⁴)R¹⁵ oder CS-NR¹⁶R¹⁷,
- R^{3a}: C₁-C₅-Alkyl, C₂-C₅-Alkenyl oder C₂-C₅-Alkinyl, wobei die drei letztgenannten Reste unsubstituiert oder durch einen oder mehrere Reste aus der Gruppe Halogen, C₁-C₄-Alkoxy, CN, Di-(C₁-C₄-Alkyl)-amino, N₃, und C₁-C₃-Alkylthio substituiert sind,
- R^{3b}: C₁-C₅-Alkyl, C₃-C₆-Cycloalkyl, C₂-C₅-Alkenyl oder C₂-C₅-Alkinyl, wobei die vier letztgenannten Reste unsubstituiert oder durch einen oder mehrere Reste aus der Gruppe Halogen, C₁-C₄-Alkoxy, CN, Di-(C₁-C₄-Alkyl)-amino, N₃, und C₁-C₃-Alkylthio substituiert sind,
- R^{4a}: CO-(C₁-C₅-Alkyl) oder CO₂-(C₁-C₅-Alkyl),
wobei die zwei letztgenannten Reste unsubstituiert oder durch einen oder mehrere Reste aus der Gruppe OH, NR¹⁸R¹⁹, N₃, CN, CO₂H, S(O)ₓ-(C₁-C₄-Alkyl) und CO₂-(C₁-C₃-Alkyl) substituiert sind,
oder Aminocarbonyl oder Aminosulfonyl, wobei die beiden letztgenannten Reste unsubstituiert oder durch gleiche oder verschiedene Reste aus der Gruppe C₁-C₄-Alkyl und C₁-C₄-Haloalkyl N-substituiert sind,
- R^{4b}: CO-(C₁-C₅-Alkyl), CO-(C₃-C₆-Cycloalkyl), CO₂-(C₁-C₅-Alkyl), CS-(C₁-C₅-Alkyl), CO-S-(C₁-C₆-Alkyl), CS-O-(C₁-C₆-Alkyl) oder CS-S-(C₁-C₆-Alkyl), wobei die sieben letztgenannten Reste unsubstituiert oder durch einen oder mehrere Reste aus der Gruppe CN, N₃, CO₂H, CO₂-(C₁-C₃-Alkyl), OH, NR²⁰R²¹, S(O)ₓ-(C₁-C₄-Alkyl), CO-NR²²R²³ und substituiert sind, oder SO₂NR²⁹R³⁰,
- R⁵: C₁-C₅-Alkyl, C₂-C₅-Alkenyl, C₂-C₅-Alkinyl, C₃-C₆-Cycloalkyl oder C₄-C₇-Cycloalkylalkyl, wobei die fünf letztgenannten Reste unsubstituiert oder durch einen oder mehrere Reste aus der Gruppe Halogen, CN, N₃, C₁-C₃-Alkoxy, C₁-C₃-Alkylthio, Amino, Mono- und Di-(C₁-C₄-alkyl)-amino, NO₂, SCN, C₁-C₃-Haloalkoxy und C₁-C₃-Haloalkylthio substituiert sind,
- R⁶: einen Rest wie R⁵,
- R⁷: einen Rest wie R⁵,
- R⁸: einen Rest wie R⁵,
- R⁹: H, C₁-C₆-Alkyl, C₂-C₆-Alkenyl, C₂-C₆-Alkinyl, C₃-C₇-Cycloalkyl, wobei die vier letztgenannten Reste unabhängig voneinander unsubstituiert oder durch einen oder mehrere Reste aus der Gruppe Halogen, C₁-C₄-Alkoxy, C₁-C₄-Alkylthio, C₁-C₄-Haloalkoxy, C₁-C₄-Haloalkylthio und CN substituiert sind,
- R¹⁰: H, C₁-C₆-Alkyl, C₂-C₆-Alkenyl, C₂-C₆-Alkinyl, wobei die drei letztgenannten Reste unsubstituiert oder durch einen oder mehrere Reste aus der Gruppe C₁-C₃-Alkoxy, C₁-C₃-Alkylthio, Halogen, CN, Amino, C₁-C₄-Alkyl-amino und Di-(C₁-C₄-alkyl)-amino substituiert sind, oder C₁-C₄-Alkoxy oder OH,
- R¹¹: H, C₁-C₆-Alkyl, C₂-C₆-Alkenyl, C₂-C₆-Alkinyl, wobei die drei letztgenannten Reste unsubstituiert oder durch einen oder mehrere Reste aus der Gruppe Halogen, CN, NH₂, Mono- und Di-(C₁-C₄-alkyl)-amino, C₁-C₃-Alkoxy und C₁-C₃-Alkylthio substituiert sind, oder
- NR¹⁰R¹¹: gemeinsam einen heterocyclischen Ring, der neben dem N-Ringatom noch bis zu zwei Heteroringatome aus der Gruppe N, O und S enthalten und weiter substituiert sein kann,
- R¹² und R¹³: unabhängig voneinander H, C₁-C₄-Alkyl, C₂-C₄-Alkenyl oder C₂-C₄-Alkinyl oder
- R¹² und R¹³: gemeinsam eine Alkylenkette mit 3 oder 4 C-Atomen, die noch mit C₁-C₄-Alkyl oder C₁-C₄-Alkoxy substituiert sein kann,
- R¹⁴: H, OH, NH₂, NHR³¹, NR³¹₂, C₁-C₄-Alkyl, C₁-C₄-Alkoxy, C₂-C₄-Alkenyl oder C₂-C₄-Alkinyl, wobei die letztgenannten vier Reste unsubstituiert oder durch einen oder mehrere Reste aus der Gruppe Halogen, C₁-C₃-Alkoxy und C₁-C₃-Alkylthio substituiert sind,
- R¹⁵: H, C₁-C₆-Alkyl, C₁-C₆-Alkoxy, C₂-C₆-Alkenyl, C₂-C₆-Alkinyl, wobei die vier letztgenannten Reste unsubstituiert oder durch einen oder mehrere Reste aus der Gruppe Halogen, C₁-C₃-Alkoxy, C₁-C₃-Alkylthio substituiert sind,
- R¹⁶: einen Rest wie R¹⁰,
- R¹⁷: einen Rest wie R¹¹,
- R¹⁸: unabhängig von R¹⁹ H, C₁-C₄-Alkyl, C₂-C₅-Alkenyl oder C₂-C₅-Alkinyl,
wobei die drei letztgenannten Reste unsubstituiert oder durch einen oder mehrere Reste aus der Gruppe Halogen, C₁-C₃-Alkoxy, C₁-C₃-Alkylthio und CN substituiert sind,
- R¹⁹: unabhängig von R¹⁸ einen Rest wie R¹⁸ oder C₁-C₃-Alkoxy oder OH oder
- NR¹⁸R¹⁹: gemeinsam einen heterocyclischen Ring, der neben dem N-Ringatom noch bis zu zwei Heteroringatome aus der Gruppe N, O und S enthalten und weiter substituiert sein kann, z. B. eine Gruppe der Formeln K1 bis K5 bedeuten, 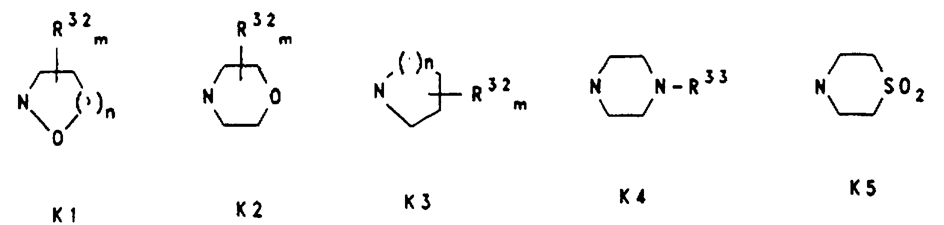
- R²⁰: unabhängig von R²¹ H, C₁-C₄-Alkyl, C₂-C₄-Alkenyl oder C₂-C₄-Alkinyl, wobei drei letztgenannten die Reste unsubstituiert oder durch einen oder mehrere Reste aus der Gruppe Halogen, CN, NH₂, Mono- und Di-(C₁-C₄)-alkylamino, C₁-C₃-Alkoxy, C₁-C₃-Alkylthio und CO₂-(C₁-C₃-Alkyl) substituiert sind,
- R²¹: unabhängig von R²⁰ einen Rest wie R²⁰ oder CHO, CO-(C₁-C₅-Alkyl) oder CO₂-(C₁-C₅-Alkyl) oder
- NR²⁰R²¹: einen heterocyclischen Ring analog NR¹⁸R¹⁹,
- R²² und R²³: unabhängig voneinander H, C₁-C₃-Alkyl, C₂-C₄-Alkenyl oder C₂-C₄-Alkinyl oder
- NR²²R²³: einen heterocyclischen Ring analog NR¹⁸R¹⁹,
- R²⁹: unabhängig von R³⁰ H, C₁-C₄-Alkyl, C₂-C₅-Alkenyl oder C₂-C₅-Alkinyl, wobei die drei letztgenannten Reste unsubstituiert oder durch einen oder mehrere Reste aus der Gruppe Halogen, C₁-C₃-Alkoxy, C₁-C₃-Alkylthio und CN substituiert sind,
- R³⁰: unabhängig von R²⁹ H, C₁-C₄-Alkyl, C₂-C₅-Alkenyl oder C₂-C₅-Alkinyl, wobei die drei letztgenannten Reste unsubstituiert oder durch einen oder mehrere Reste aus der Gruppe Halogen, C₁-C₃-Alkoxy, C₁-C₃-Alkylthio und CN substituiert sind, oder C₁-C₃-Alkoxy oder OH oder
- NR²⁹R³⁰: einen heterocyclischen Ring analog NR¹⁸R¹⁹,
- R³¹: C₁-C₄-Alkyl, C₂-C₄-Alkenyl oder C₂-C₄-Alkinyl, wobei die drei letztgenannten Reste unabhängig voneinander unsubstituiert oder durch einen oder mehrere Reste aus der Gruppe Halogen, C₁-C₄-Alkoxy und C₁-C₄-Alkylthio substituiert sind,
- R³²: H, C₁-C₃-Alkyl, C₁-C₃-Haloalkyl, C₁-C₃-Alkoxyalkyl, Halogen oder CN,
- R³³: H, C₁-C₃-Alkyl, C₂-C₄-Alkenyl, C₂-C₄-Alkinyl, wobei die drei letztgenannten Reste unsubstituiert oder durch einen Rest aus der Gruppe Halogen, C₁-C₄-Alkoxy, CN und C₁-C₄-Alkylthio substituiert ist,
- x: - unabhängig von den übrigen Indices x - 0, 1 oder 2,
- n: - unabhängig von den übrigen Indices n - 1, 2, 3 oder 4
- m: - unabhängig von den übrigen Indices m - 1 oder 2,
- W: O oder S, vorzugsweise 0,
- X, Y: unabhängig voneinander Wasserstoff, Halogen, C₁-C₂-Alkyl, C₁-C₂-Alkoxy oder C₁-C₂-Alkylthio, wobei die drei letztgenannten Reste unsubstituiert oder durch einen oder mehrere Reste aus der Gruppe Halogen, C₁-C₂-Alkoxy und C₁-C₂-Alkylthio substituiert sind, oder Mono- oder Di(C₁-C₂-alkyl)amino, C₃-C₄-Alkenyl, C₃-C₄-Alkenyloxy oder C₃-C₄-Alkinyloxy und
- Z: bedeutet CH oder N, vorzugsweise CH.

In der Formel (I) bzw. in den folgenden Formeln können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die Kohlenstoffgerüste mit 1 bis 4 C-Atomen, bzw. bei ungesättigten Gruppen mit 2 bis 4 C-Atomen bevorzugt. Alkylreste, auch in den zusammengesetzten Bedeutungen wie Alkoxy, Haloalkyl usw., bedeuten z. B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste, Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Aliphatischer Rest bedeutet einen gesättigten oder ungesättigten, acyclischen oder cyclischen Kohlenwasserstoffrest, der durch Heteroatome wie O, S und N unterbrochen und weitere funktionelle Gruppen aufweisen kann, z.B. Nitro, Cyano, Carboxy, Alkylcarbonyl, Alkoxycarbonyl, Carbonamid etc.

Ein unsubstituierter oder substituierter Kohlenwasserstoffrest ist beispielsweise Alkyl, Alkenyl, Alkinyl, Cycloalkyl, Cycloalkenyl, Aryl (inklusive Heteroaryl) wie Phenyl, Naphthyl, Pyridyl und Thienyl, Aralkyl wie Benzyl, Alkylaryl wie Tolyl, wobei die genannten Reste unsubstituiert oder durch einen oder mehrere Reste substituiert sind, beispielsweise durch Reste aus der Gruppe Halogen, Alkoxy, CN, Amino, Mono- und Dialkylamino, Azido, Alkylthio, gegebenenfalls substituiertes Carbonamido, Nitro, Alkanoyl, Carboxy, Alkoxycarbonyl, Alkylsulfinyl, Alkylsulfonyl und andere vorzugsweise niedermolekulare Reste oder funktionelle Gruppen, die in der betreffenden Verbindung bei Raumtemperatur relativ stabil sind und unter wäßrig neutralen Bedingungen keine oder nur geringe Reaktionsfähigkeit aufweisen.

Substituiertes Aminocarbonyl oder Aminosulfonyl bedeutet beispielsweise einen betreffenden N-monosubstituierten oder N,N-disubstituierten Rest, dessen Substituenten gegebenenfalls verschiedene Reste aus der Gruppe Alkyl, Haloalkyl, Aryl (inklusive Heteroaryl), wie gegebenenfalls substituiertes Phenyl, und Acyl, z. B. Alkanoyl wie Acetyl und Arylcarbonyl wie Benzoyl, vorzugsweise Alkyl.

Ein über ein N-Atom gebundener heterocyclischer Rest, der noch weiter substituiert sein kann, ist beispielsweise 1-Piperidinyl, 1-Piperazinyl, 4-Morpholinyl, 1-Pyrrolidinyl, 1-Oxa-3-aza-cyclopent-3-yl, 1-Oxa-2-aza-cyclopent-2-yl, wobei diese Reste gegebenenfalls durch weitere Reste, z. B. Alkyl, Alkoxy und Halogen, substituiert sind.

Gegebenenfalls substituiertes Phenyl entspricht unsubstituiertes Phenyl oder substituiertes Phenyl, wobei die Substituenten beispielsweise einen oder mehrere, vorzugsweise 1, 2 oder 3 Reste aus der Gruppe Halogen, Alkyl, Haloalkyl, Alkoxy, Haloalkoxy, Nitro, Cyano, Alkoxycarbonyl, Alkylcarbonyl, Alkanoyl, Carbamoyl, Mono- und Dialkylaminocarbonyl, Mono- und Dialkylamino, Alkylsulfinyl und Alkylsulfonyl bedeuten und bei Resten mit C-Atomen solche mit 1 bis 4 C-Atomen, insbesondere 1 oder 2 C-Atomen, bevorzugt sind; bevorzugt sind in der Regel Substituenten aus der Gruppe Halogen, wie Fluor und Chlor, C₁-C₄-Alkyl, vorzugsweise Methyl oder Ethyl, C₁-C₄-Haloalkyl, vorzugsweise Trifluormethyl, C₁-C₄-Alkoxy, vorzugsweise Methoxy oder Ethoxy, C₁-C₄-Haloalkoxy, Nitro und Cyano; besonders bevorzugt sind dabei Methyl, Methoxy und Chlor.

Halogen bedeutet Fluor, Chlor, Brom oder lod. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl. Entsprechendes gilt für Haloalkenyl und andere durch Halogen substituierte Reste.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in den allgemeinen Formeln (I) nicht gesondert angegeben sind. Die durch ihre spezifische Raumform definierten möglichen Stereoisomeren, wie Enantiomere, Diastereomere, Z- und E-lsomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Die Verbindungen der Formel (I) können Salze bilden, bei denen der Wasserstoff der -SO₂-NH-Gruppe oder auch andere acide Wasserstoffatome (z.B. aus COOH u.a.) durch ein für die Landwirtschaft geeignetes Kation ersetzt wird. Diese Salze sind beispielsweise Metall-, insbesondere Alkali- oder Erdalkalisalze, z. B. Natrium- und Kaliumsalze, oder auch Ammoniumsalze oder Salze mit organischen Aminen. Ebenso kann Salzbildung durch Anlagerung einer Säure an basische Gruppen, wie z.B. Amino und Alkylamino, erfolgen. Geeignete Säuren hierfür sind starke anorganische und organische Säuren, beispielsweise HCI, HBr, H₂SO₄ oder HNO₃.

Von besonderem Interesse sind erfindungsgemäße Verbindungen der Formel (I) und deren Salze, worin ein oder mehrere der folgenden Bedeutungen für die Reste R¹ bis Z gelten:

Von besonderem Interesse sind auch erfindungsgemäße Verbindungen der Formel (I) und deren Salze, worin
- G: ein Rest der Formel (G1) ist und
- R¹: H oder C₁-C₃-Alkyl, vorzugsweise H oder Methyl,
- R²: CO-OR⁵, CS-SR⁶, CO-SR⁷, CS-OR⁸, CO-R⁹, CO-NR¹⁰R¹¹, CO-O-N=CR¹²R¹³, C(=NR¹⁴)R¹⁵ oder CS-NR¹⁶R¹⁷, vorzugsweise CO-OR⁵,
- R^{3a}: C₁-C₄-Alkyl, C₁-C₄-Haloalkyl, C₂-C₄-Alkenyl oder C₂-C₄-Alkinyl,
- R^{4a}: CO-(C₁-C₄-Alkyl), CO₂-(C₁-C₅-Alkyl), CO-NH₂, CO-NH(C₁-C₄-Alkyl), CO-N(C₁-C₄-Alkyl)₂ oder SO₂-NH₂, SO₂-NH(C₁-C₄-Alkyl) oder SO₂-N(C₁-C₄-Alkyl)₂,
vorzugsweise Acetyl, Propionyl, Methoxycarbonyl, Ethoxycarbonyl, Propoxycarbonyl, Isopropoxycarbonyl, Mono- oder Dimethylaminocarbonyl, Mono- oder Diethylaminocarbonyl, Mono- oder Dipropylaminocarbonyl oder Mono- oder Diisopropylaminocarbonyl,
- R⁵: C₁-C₄-Alkyl, C₁-C₄-Haloalkyl, C₂-C₄-Alkenyl, C₂-C₄-Alkinyl, C₃-C₆-Cycloalkyl oder C₄-C₇-Cycloalkylmethyl,
- R⁶: einen Rest wie R⁵,
- R⁷: einen Rest wie R⁵,
- R⁸: einen Rest wie R⁵,
- R⁹: H, C₁-C₄-Alkyl, C₂-C₄-Alkenyl, C₂-C₄-Alkinyl oder C₃-C₆-Cycloalkyl, wobei die vier letztgenannten Reste unabhängig voneinander unsubstituiert oder durch einen oder mehrere Reste aus der Gruppe Halogen, C₁-C₂-Alkoxy, C₁-C₂-Alkylthio, C₁-C₂-Haloalkoxy, C₁-C₂-Haloalkylthio und CN substituiert sind,
- R¹⁰ und R¹¹: unabhängig voneinander H, C₁-C₄-Alkyl, C₂-C₄-Alkenyl oder C₂-C₄-Alkinyl,
- R¹² und R¹³: unabhängig voneinander H, C₁-C₄-Alkyl, C₂-C₄-Alkenyl oder C₂-C₄-Alkinyl oder
- R¹² und R¹³: gemeinsam eine Alkylenkette mit 3 oder 4 C-Atomen, die noch mit C₁-C₄-Alkyl oder C₁-C₄-Alkoxy substituiert sein kann,
- R¹⁴: NH₂, NHR³¹, NR³¹₂, C₁-C₄-Alkyl, C₁-C₄-Alkoxy, C₂-C₄-Alkenyl oder C₂-C₄-Alkinyl,
- R¹⁵: H, C₁-C₄-Alkyl, C₁-C₄-Alkoxy, C₂-C₄-Alkenyl oder C₂-C₄-Alkinyl,
- R¹⁶: einen Rest wie R¹⁰,
- R¹⁷: einen Rest wie R¹¹,
- R³¹: C₁-C₄-Alkyl, C₂-C₄-Alkenyl oder C₂-C₄-Alkinyl,
- W: O oder S, vorzugsweise O,
- X, Y: unabhängig voneinander Halogen, C₁-C₂-Alkyl, C₁-C₂-Haloalkyl, C₁-C₂-Alkoxy, C₁-C₂-Haloalkoxy, C₁-C₂-Alkylthio, Mono- oder Di(C₁-C₂-alkyl)amino, C₃-C₄-Alkenyl, C₃-C₄-Alkenyloxy oder C₃-C₄-Alkinyloxy, vorzugsweise der eine Rest Methyl, Methoxy oder Chlor und der andere Rest Methyl oder Methoxy, und
- Z: CH oder N, vorzugsweise CH bedeuten.

Von besonderem Interesse sind auch erfindungsgemäße Verbindungen der Formel (I) und deren Salze, worin
- G: ein Rest der Formel (G3) ist und
- R¹: H oder C₁-C₃-Alkyl, vorzugsweise H oder Methyl,
- R²: CO-OR⁵, CS-SR⁶, CO-SR⁷, CS-OR⁸, CO-R⁹, CO-NR¹⁰R¹¹, CO-O-N=CR¹²R¹³, C(=NR¹⁴)R¹⁵ oder CS-NR¹⁶R¹⁷, vorzugsweise CO-OR⁵,
- R^{3b}: C₁-C₄-Alkyl, C₃-C₈-Cycloalkyl, C₁-C₄-Haloalkyl, C₂-C₄-Alkenyl oder C₂-C₄-Alkinyl,
- R^{4b}: CO-(C₁-C₄-Alkyl), CO-(C₃-C₆-Cycloalkyl) oder CO₂-(C₁-C₄-Alkyl), wobei die 3 letztgenannten Reste unsubstituiert oder durch einen oder mehrere Reste aus der Gruppe CN, NR²⁰R²¹, S(O)ₓ-(C₁-C₄-Alkyl) und CO-NR²²R²³ substituiert sind,
oder SO₂NR²⁹R³⁰,
- R⁵: C₁-C₄-Alkyl, C₂-C₄-Alkenyl, C₂-C₄-Alkinyl, C₃-C₆-Cycloalkyl oder C₄-C₇-Cycloalkylmethyl, wobei die fünf letztgenannten Reste unsubstituiert oder durch einen oder mehrere Reste aus der Gruppe Halogen, C₁-C₂-Alkoxy und C₁-C₂-Haloalkoxy substituiert sind,
- R⁶: einen Rest wie R⁵,
- R⁷: einen Rest wie R⁵,
- R⁸: einen Rest wie R⁵,
- R⁹: H, C₁-C₄-Alkyl, C₂-C₄-Alkenyl, C₂-C₄-Alkinyl oder C₃-C₆-Cycloalkyl, wobei die vier letztgenannten Reste unabhängig voneinander unsubstituiert oder durch einen oder mehrere Reste aus der Gruppe Halogen, C₁-C₂-Alkoxy, C₁-C₂-Alkylthio, C₁-C₂-Haloalkoxy, C₁-C₂-Haloalkylthio und CN substituiert sind,
- R¹⁰ und R¹¹: unabhängig voneinander H, C₁-C₄-Alkyl, C₂-C₄-Alkenyl oder C₂-C₄-Alkinyl,
- R¹² und R¹³: unabhängig voneinander H, C₁-C₄-Alkyl, C₂-C₄-Alkenyl oder C₂-C₄-Alkinyl oder
- R¹² und R¹³: gemeinsam eine Alkylenkette mit 3 oder 4 C-Atomen, die noch mit C₁-C₄-Alkyl oder C₁-C₄-Alkoxy substituiert sein kann,
- R¹⁴: NH₂, NHR³¹, NR³¹₂, C₁-C₄-Alkyl, C₁-C₄-Alkoxy, C₂-C₄-Alkenyl oder C₂-C₄-Alkinyl,
- R¹⁵: H, C₁-C₄-Alkyl, C₁-C₄-Alkoxy, C₂-C₄-Alkenyl oder C₂-C₄-Alkinyl,
- R¹⁶: einen Rest wie R¹⁰,
- R¹⁷: einen Rest wie R¹¹,
- R²⁰: unabhängig von R²¹ H, C₁-C₄-Alkyl, C₂-C₄-Alkenyl oder C₂-C₄-Alkinyl, wobei die drei letzgenannten Reste unsubstituiert oder durch einen oder mehrere Reste aus der Gruppe Halogen und C₁-C₂-Alkoxy substituiert sind,
- R²¹: unabhängig von R²⁰ einen Rest wie R²⁰ oder CHO, CO-(C₁-C₄-Alkyl) oder CO₂-(C₁-C₄-Alkyl) oder
- NR²⁰R²¹: gemeinsam einen heterocyclischen Ring, der neben dem N-Ringatom noch ein Heteroringatom aus der Gruppe N, O und S enthalten und weiter durch Reste aus der Gruppe C₁-C₄-Alkyl, C₁-C₄-Alkoxy und Halogen substituiert und, im Falle von S als Heteroringatom, am S-Atom oxidiert sein kann, z. B. eine Gruppe der Formeln K1 bis K5 bedeuten, 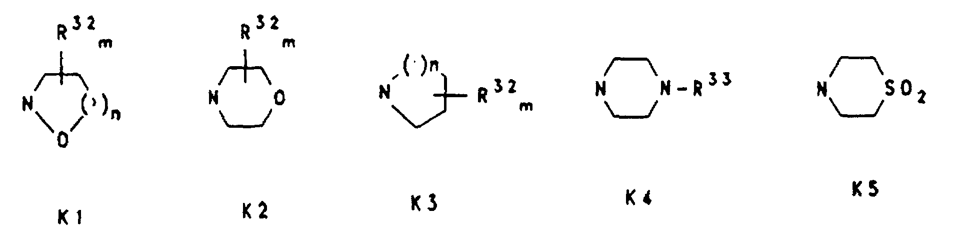
- R²² und R²³: unabhängig voneinander H, C₁-C₃-Alkyl, C₂-C₄-Alkenyl oder C₂-C₄-Alkinyl oder
- NR²²R²³: einen heterocyclischen Ring analog NR²⁰R²¹,
- R²⁹: unabhängig von R³⁰ H, C₁-C₄-Alkyl, C₂-C₄-Alkenyl, C₂-C₄-Alkinyl, wobei die drei letztgenannten Reste unsubstituiert oder durch einen oder mehrere Reste aus der Gruppe Halogen, C₁-C₃-Alkoxy, C₁-C₃-Alkylthio und CN substituiert sind,
- R³⁰: unabhängig von R²⁹ H, C₁-C₄-Alkyl, C₂-C₄-Alkenyl, C₂-C₄-Alkinyl, wobei die drei letztgenannten Reste unsubstituiert oder durch einen oder mehrere Reste aus der Gruppe Halogen, C₁-C₃-Alkoxy, C₁-C₃-Alkylthio und CN substituiert sind, oder C₁-C₃-Alkoxy oder OH oder
- NR²⁹R³⁰: einen heterocyclischen Ring analog NR²⁰R²¹,
- R³¹: C₁-C₄-Alkyl, C₂-C₄-Alkenyl oder C₂-C₄-Alkinyl,
- R³²: H, C₁-C₃-Alkyl, C₁-C₃-Haloalkyl, C₁-C₃-Alkoxyalkyl, Halogen oder CN,
- R³³: H, C₁-C₃-Alkyl, C₂-C₄-Alkenyl, C₂-C₄-Alkinyl, wobei die drei letztgenannten Reste unsubstituiert oder durch einen Rest aus der Gruppe Halogen, C₁-C₂-Alkoxy und C₁-C₂-Alkylthio substituiert sind,
- x: - unabhängig von den übrigen Indices x - 0, 1 oder 2,
- n: - unabhängig von den übrigen Indices n - 1, 2 oder 3
- m: - unabhängig von den übrigen Indices m - 1 oder 2,
- W: O oder S, vorzugsweise O,
- X, Y: unabhängig voneinander Halogen, C₁-C₂-Alkyl, C₁-C₂-Haloalkyl, C₁-C₂-Alkoxy, C₁-C₂-Haloalkoxy, C₁-C₂-Alkylthio, Mono- oder Di(C₁-C₂-alkyl)amino, C₃-C₄-Alkenyl, C₃-C₄-Alkenyloxy oder C₃-C₄-Alkinyloxy, vorzugsweise der eine Rest Methyl, Methoxy oder Chlor und der andere Rest Methyl oder Methoxy, und
- Z: CH oder N, vorzugsweise CH bedeuten.

Weiterer Gegenstand der vorliegenden Erfindung sind Verfahren zur Herstellung der erfindungsgemäßen Verbindungen der Formel (1) oder deren Salze, dadurch gekennzeichnet, daß man
a) eine Verbindung der Formel (II) 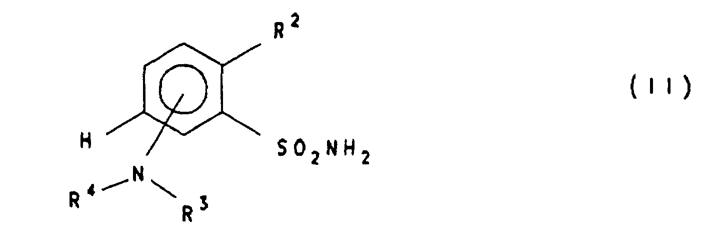 mit einem heterocyclischen Carbamat der Formel (III), 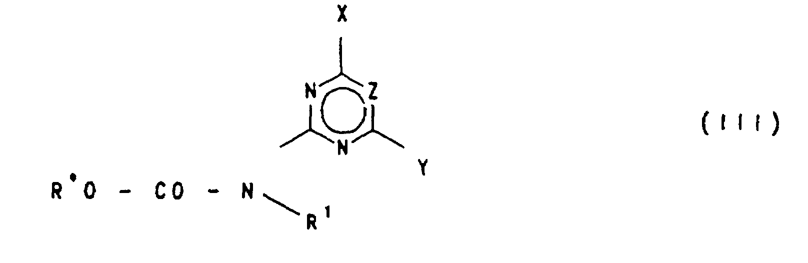 worin R* gegebenenfalls substituiertes Phenyl oder C₁-C₄-Alkyl bedeuten, umsetzt oder
b) ein Sulfonylisocyanat der Formel (IV) 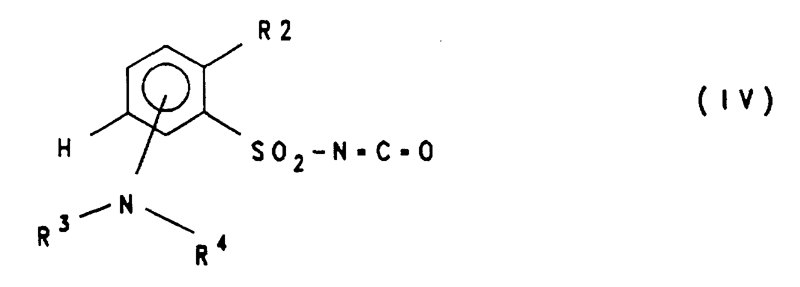 mit einem Aminoheterocyclus der Formel (V) 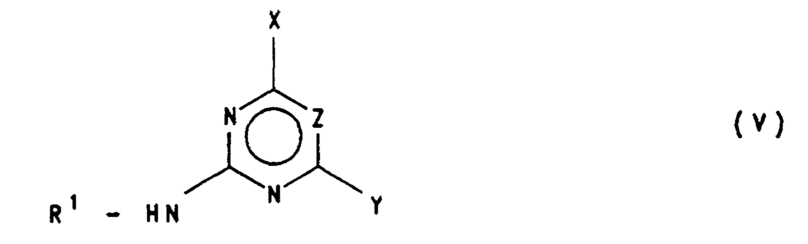 umsetzt, oder
c) ein Sulfochlorid der Formel (VI) 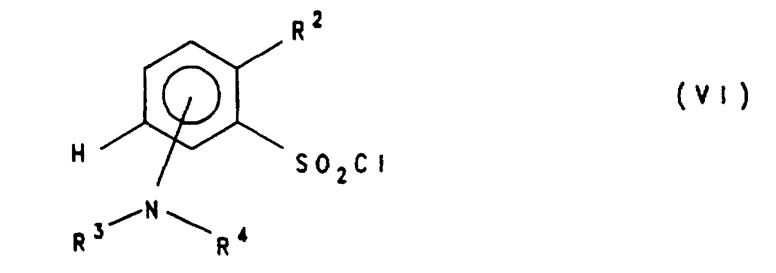 mit einem Aminoheterocyclus der genannten Formel (V) in Gegenwart eines Isocyanat-Salzes, z. B. eines Na- oder K-Salzes, umsetzt oder
d) ein Sulfonamid der genannten Formel (II) mit einem (Thio-)lsocyanat der Formel (XIX) 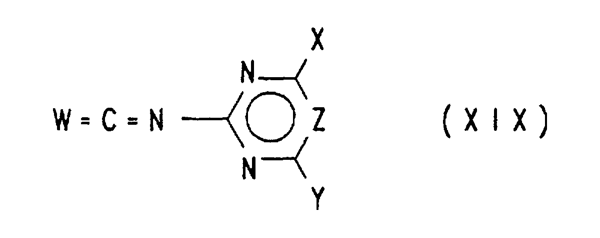
in Gegenwart einer Base, z.B. Kaliumcarbonat oder Triethylamin, umsetzt,
wobei in den obigen Formeln (II) bis (VI) und (XIX) die R¹, R², W, X, Y und Z wie in Formel (I) definiert sind und, im Falle von Verbindungen der Formel (I) mit G = (G1), R³ und R⁴ die Bedeutung und Position wie R^{3a} bzw. R^{4a} in Formel (G1) haben und, im Falle von Verbindungen der Formel (I) mit G = (G3), R³ und R⁴ die Bedeutung und Position wie R^{3b} bzw. R^{4b} in Formel (G3) haben.

Die Sulfonamide (II), die Sulfonylisocyanate (IV) und die Sulfonylchloride (VI) sind neue Verbindungen. Sie und ihre Herstellung sind ebenfalls Gegenstand der Erfindung.

Die Umsetzung der Verbindungen der Formel (II) und (III) erfolgt vorzugsweise basenkatalysiert in inerten Solventien, wie z. B. Dichlormethan, Acetonitril, Dioxan oder THF, bei Temperaturen von -10° C bis zum Siedepunkt des jeweiligen Lösungsmittels. Als Basen werden dabei beispielsweise organische Aminbasen wie 1,8-Diazabicyclo[5.4.0]undec-7-en (DBU), insbesondere bei R* = (subst.) Phenyl (vgl. EP-A-44807), oder Trimethyl- oder Triethylaluminium, letztere insbesondere bei R* = Alkyl (vgl. EP-A-166 516), verwendet.

Zur Synthese der Verbindungen der Formel (II) stehen verschiedene Alternativen zur Verfügung:

Ausgehend von einer Verbindung der Formel (VII), beispielsweise 2-Methyl-4-nitro-benzolsulfonsäure, führt die Oxidation der Methylgruppe mit geeigneten Oxidationsmitteln, wie z.B. Kaliumpermanganat, zu den entsprechenden Carbonsäuren [analog Org. Syn. Coll. Vol. 3, 740 (1955)], welche anschließend durch säurekatalysierte Veresterung [vgl. hierzu: Tetrahedron, 36, 2409 (1980)] mit den entsprechenden Alkoholen R⁵-OH in die Ester der Formel (VIII) überführt werden (Schema 1).

Die Sulfonsäuren (VIII) können nach bekannten Standard-Methoden in die N-tert.-Butylsulfonamide (IX) transformiert werden: Hierzu werden z. B. die Kaliumsalze der Sulfonsäuren (VIII) mit Phosphoroxychlorid oder Thionylchlorid 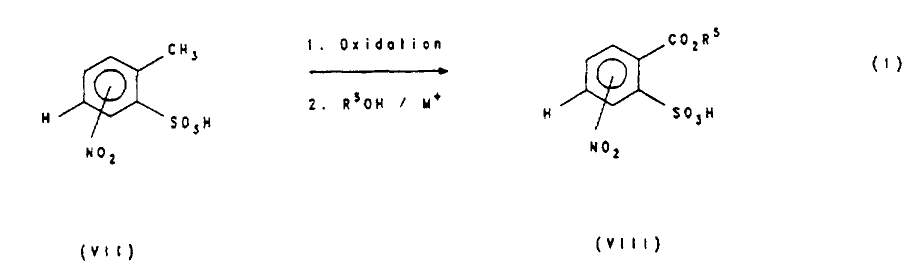 in inerten Solventien wie z. B. Acetonitril und/oder Sulfolan oder in Substanz durch Erhitzen zum Rückfluß in die entsprechenden Sulfochloride überführt (vgl. Houben-Weyl-Klamann, "Methoden der organischen Chemie", 4. Aufl., Bd. E XI/2, S. 1067-1073, Thieme Verlag Stuttgart, 1985). Anschließend werden diese Sulfochloride mit tert.-Butylamin zu den Sulfonamiden der Formel (IX) umgesetzt (Schema 2). Diese Reaktionen werden in der Regel bei Temperaturen zwischen -78° C und +80° C, vorzugsweise zwischen 0° C und 30° C, in Solventien wie Dichlormethan, Trichlormethan, THF, Dioxan, Methanol oder Ethanol durchgeführt.

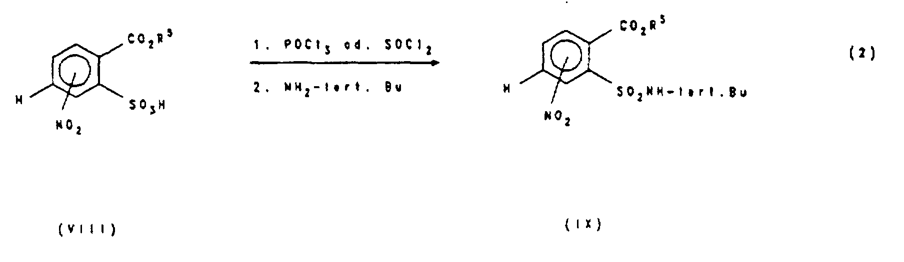

Die Nitrogruppe in der Verbindung der Formel (IX) wird anschließend zur Aminogruppe reduziert (Schema 3).

Die Reduktion kann beispielsweise mit Wasserstoff in Gegenwart eines Katalysators wie Pd oder mit Eisen in essigsaurem Medium erfolgen [vgl. hierzu: H. Berrie, G.T. Neuhold, F.S. Spring, J. Chem. Soc. 1952, 2042; M. Freifelder, "Catalytic Hydrogenation in Organic Synthesis: Procedures and Commentary", J. Wiley and Sons, New York (1978), Kap. 5].

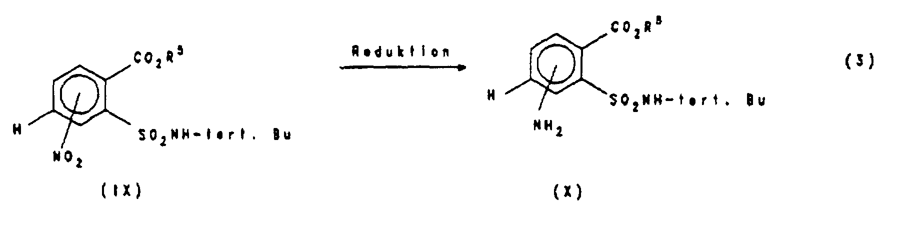

Die erhaltenen Aniline der Formel (X) werden dann an der Aminogruppe funktionalisiert: Für den Fall R³ gleich H erfolgt die Acylierung, für den Fall R³ ungleich H eine Monoalkylierung sowie zusätzlich eine Acylierung (Schema 4).

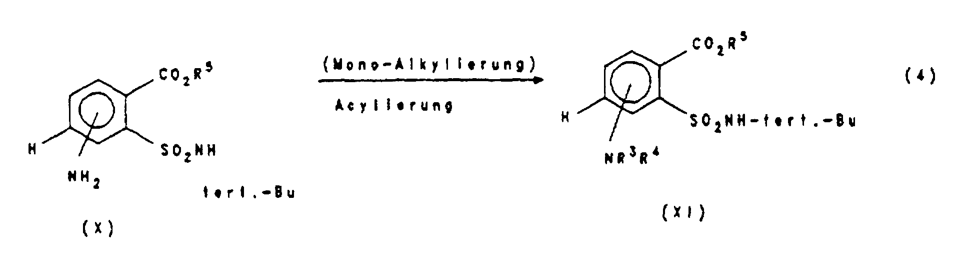

Die Monoalkylierung der NH₂-Gruppe der Verbindungen (X) läßt sich bequem nach einer Methode von S. Krishnamurthy [Tetrahedron Lett. 23, 3315 (1982)] realisieren. Die Umsetzung mit geeigneten Elektrophilen, wie z. B. Säurechloriden, Säureanhydriden, Isocyanaten, Thioisocyanat, Sulfochloriden, Amidosulfochloriden zu den N-acylierten Verbindungen (XI) kann analog literaturbekannten Verfahren realisiert werden [vgl. hierzu: A.L.J. Beckniter in J. Zabicky, "The Chemistry of Amides", S. 73-185, Interscience, New York, 1970; E. J. Corey et. al., Tetrahedron Lett. 1978, 1051; H. J. Saunders, R. J. Slocombe, Chem. Rev. 43, 203 (1948); S. Ozaki, Chem. Rev. 72, 457, 469 (1972); G. Zoelss, Arzneim.-Forsch. 33, 2 (1983); Houben-Weyl-Hagemann, "Methoden der Organischen Chemie", 4. Aufl., Bd. E4, S. 485 ff., Thieme Verlag Stuttgart, 1983; J. Golinsky, M. Mahosza, Synthesis 1978, 823; Houben-Weyl-Müller, "Methoden der organischen Chemie", 4. Aufl., Bd. IX, S. 338-400 und 605-622, Thieme Verlag Stuttgart 1955; Houben-Weyl-Klamann, "Methoden der organischen Chemie", 4. Aufl., Bd. E11/2, S. 1020-22, Thieme Verlag Stuttgart, 1985]

Die Abspaltung der tert.-Butylgruppe aus den Verbindungen der Formel (XI) zu den Sulfonamiden (II) erfolgt z. B. mit starken Säuren (vgl. hierzu WO-89/10921) (Schema 5).

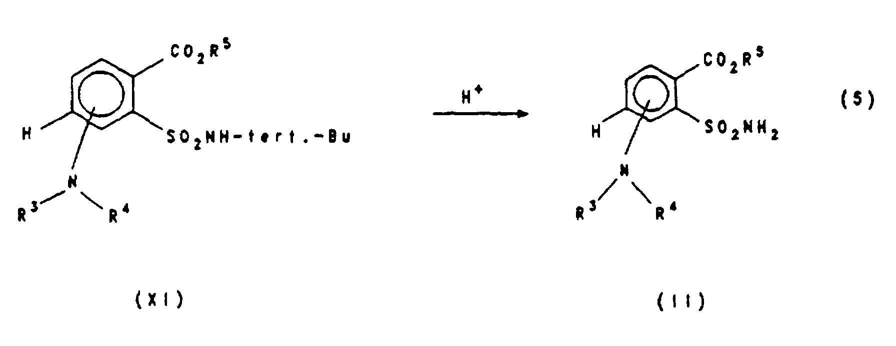

Als starke Säuren kommen z.B. Mineralsäuren wie Schwefelsäure oder Salzsäure, oder starke organische Säuren, wie Trifluoressigsäure, in Frage. Die Umsetzungen werden beispielsweise bei Temperaturen von -20°C bis zur jeweiligen Rückflußtemperatur, vorzugsweise bei 0°C bis 40°C, in einem inerten Solvens, wie z.B. Dichlormethan oder Trichlormethan, oder in Substanz duchgeführt.

Einen weiteren Zugang zu den Verbindungen (II) bietet die folgende Reaktionssequenz (vgl. Schema 6):

Ausgehend von Amino-nitrotoluolen der Formel (XII), z. B. 3-Amino-2-methyl-nitrobenzol, werden die Substituenten R³ und R⁴ durch Monoalkylierung und Acylierung eingeführt (analog Schema 4). Die auf diese Art erhaltenen Verbindungen (XIII) werden einer Oxidation - z.B. mit Kaliumpermanganat - zu den Carbonsäuren unterworfen (analog Schema 1). Diese Benzoesäure-Derivate werden nun nach bekannten Standardverfahren verestert (analog Schema 1). Die erhaltenen aromatischen Ester (XIV) können zu den Anilinen der Formel (XV) reduziert werden (analog Schema 3). Nach der Diazotierung der Aniline (XV) und anschließender Umsetzung mit Schwefeldioxid werden die Sulfochloride (VI) gewonnen (Houben-Weyl-Müller, "Meth. d. org. Chemie", 4. Aufl., Bd. IX, S. 563 ff., Thieme Verlag Stuttgart 1955).
Die Ammonolyse der Sulfochloride (VI) führt letztlich zu den Sulfonamiden (II). Diese Reaktion erfolgt beispielsweise bei Temperaturen zwischen 0°C und 40°C in Gegenwart organischer Solventien wie Tetrahydrofuran, Dioxan, Dichlormethan, Trichlormethan, Methanol, Ethanol etc.

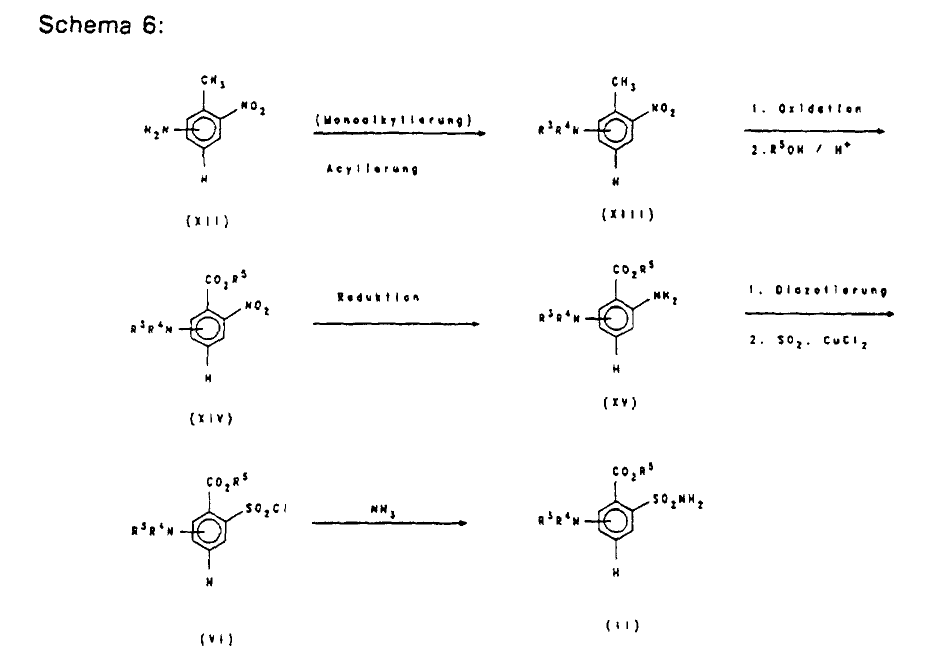

Einen alternativen Zugang zu den Suffochloriden (VI) ermöglicht die folgende Reaktionssequenz (Schema 7):

Die Nitrobenzoesäuren der Formel (XVI), z. B. 2-Chlor-3-nitro- oder 2-Chlor-5-nitro-benzoesäure, werden nach der Veresterung (analog Schema 1, 2. Schritt) mit dem Salz eines geeigneten Mercaptans bei Temperaturen zwischen 0°C und 80°C in inerten organischen Solventien - wie z.B. Acetonitril, Methanol oder Ethanol - zu den Sulfiden der Formel (XVII) umgesetzt. Als Mercaptane eignen sich beispielsweise die Natrium- bzw. Kaliumsalze von Benzylmercaptan.
Nach der Reduktion der Nitrogruppe der Verbindung (XVII) sowie der Einführung der Substituenten R³ und R⁴ (analog Schema 4) zu der Verbindung der Formel (XVIII) wird das Sulfid mit Chlor oder Hypochlorit oxidativ zu den Sulfonylchloriden (VI) gespalten (vgl. hierzu: R.T. Langler, Can. J. Chem. 54, 498 (1976); Houben-Weyl-Mueller, "Methoden der Organischen Chemie", 4. Aufl. Bd. 9, S. 580 - 583, Thieme Verlag Stuttgart, 1955); diese Umsetzungen erfolgen zwischen -10°C und 60°C - bevorzugt zwischen 0°C und 15°C - im Zweiphasensystem. Als wäßrige Phase eignen sich beispielsweise Wasser, Phosphatpuffer-Lösungen (pH = 7) oder Salzsäure, als organische z.B. Di- oder Trichlormethan.

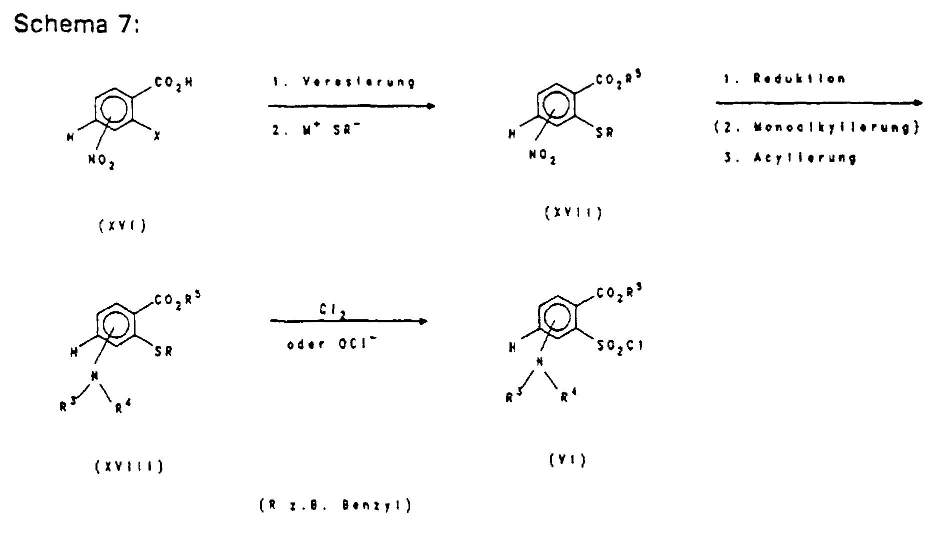

Die für die Umsetzung der Verbindungen (II) nach Variante a) benötigten Carbamate der Formel (III) sind literaturbekannt oder lassen sich analog bekannten Verfahren herstellen (vgl. EP-A-70 804 oder US-A-4,480,101).

Die Phenylsulfonylisocyanate der Formel (IV) lassen sich z.B. analog den Verfahren aus EP-A-184 385 aus Verbindungen der Formel (II), z.B. mit Phosgen, herstellen.
Die Umsetzung der Verbindungen (IV) mit den Aminoheterocyclen der Formel (V) führt man vorzugsweise in inerten, aprotischen Lösungsmitteln, wie z.B. Dioxan, Acetonitril oder Tetrahydrofuran bei Temperaturen zwischen 0°C und der Siedetemperatur des Lösungsmittels durch.

Die Umsetzung der Sulfochloride (VI) mit den Aminoheterocyclen der Formel (V) und Isocyanat-Salzen wie Natriumisocyanat und Kaliumisocyanat erfolgt z. B. in aprotischen Solventien, wie z.B. Acetonitril, in Gegenwart von 0,5 bis 2 Äquivalenten Base bei Temperaturen zwischen -10°C und 60°C, vorzugsweise bei 15°C bis 40°C. Als Base kommen z.B. Pyridin, Picolin oder Lutidin oder eine Mischung aus diesen in Betracht (vgl. US-A-5,157,119).

Die (Thio-)lsocyanate der Formel (XIX) sind nach literaturbekannten Verfahren erhältlich (EP-A-232067, EP-A-166516). Die Umsetzung der (Thio-)Isocyanate (XIX) mit Verbindungen (II) erfolgt bei -10 °C bis 100 °C, vorzugsweise 20 bis 100 °C, in einem inerten aprotischen Lösungsmittel, wie z. B. Aceton oder Acetonitril, in Gegenwart einer geeigneten Base, z. B. N(C₂H₅)₃ oder K₂CO₃.

Die Salze der Verbindungen der Formel (I) werden vorzugsweise in inerten Lösungsmitteln wie z. B. Wasser, Methanol, Aceton, Dichlormethan, Tetrahydrofuran, Toluol oder Heptan, bei Temperaturen von 0 bis 100° C hergestellt. Geeignete Basen zur Herstellung der erfindungsgemäßen Salze sind beispielsweise Alkalicarbonate, wie Kaliumcarbonat, Alkali- und Erdalkalihydroxide, wie NaOH, KOH und Ca(OH)₂, Ammoniak oder eine geeignete Aminbase, wie Triethylamin oder Ethanolamin. Als Säuren zur Salzbildung eignen sich z.B. HCI, HBr, H₂SO₄ oder HNO₃.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die erfindungsgemäßen Verbindungen der Formel (I) oder deren Salze weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im einzelnen seien beispielsweise einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.
Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung, z.B. durch Auslösen von Desikkation und Wuchstauchung, eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) oder deren Salze enthalten.

Die Verbindungen der Formel (I) oder deren Salze können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, anderen Herbiziden, Fungiziden, Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden:
Alkylarylsulfonsaure Calcium-Salze wie
Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z. B. Sorbitanfettsäureester oder Polyoxethylensorbitanester wie z. B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z. B. oben bei den anderen Formulierungstypen bereits aufgeführt sind, hergestellt werden.

Emulsionen, z. B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z. B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Die agrochemischen Zubereitungen enthalten in der Regel 0,1 bis 99 Gew.-%, insbesondere 0,1 bis 95 Gew.-%, Wirkstoff der Formel (I) oder deren Salze.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30, vorzugsweise meistens 5 bis 20 Gew.-% an Wirkstoff. versprühbare Lösungen etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Wirkstoffe in Mischungsformulierungen oder im Tank-Mix sind beispielsweise bekannte Wirkstoffe einsetzbar, wie sie in z.B. in Weed Research 26, 441-445 (1986), oder "The Pesticide Manual", 9th edition, The British Crop Protection Council, 1990/91, Bracknell, England, und dort zitierter Literatur beschrieben sind. Als literaturbekannte Herbizide, die mit den Verbindungen der Formel (I) kombiniert werden können, sind z. B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d. h. [[[7-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim; ametryn; amidosulfuron; amitrol; AMS, d. h. Ammoniumsulfamat; anilofos; asulam; atrazin; aziprotryn; barban; BAS 516 H, d. h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; carbetamide; CDAA, d. h. 2-Chlor-N,N-di-2-propenylacetamid; CDEC, d. h. Diethyldithiocarbaminsäure-2-chlorallylester; CGA 184927, d. h. 2-[4-[(5-Chlor-3-fluor-2-pyridinyl)-oxy]-phenoxy]-propansäure und 2-propynylester; chlomethoxyfen; chloramben; chlorazifop-butyl, pirifenop-butyl; chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clomazone; clomeprop; cloproxydim; clopyralid; cyanazine; cycloate; cycloxydim; cycluron; cyperquat; cyprazine; cyprazole; 2,4-DB; dalapon; desmedipham; desmetryn; di-allate; dicamba; dichlobenil; dichlorprop; diclofop-methyl; diethatyl; difenoxuron; difenzoquat; diflufenican; dimefuron; dimethachlor; dimethametryn; dimethazone, clomazon; dimethipin; dimetrasulfuron, cinosulfuron; dinitramine; dinoseb; dinoterb; diphenamid; dipropetryn; diquat; dithiopyr; diuron; DNOC; eglinazine-ethyl; EL 177, d. h. 5-Cyano-1-(1,1-dimethylethyl)-N-methyl-3H-pyrazole-4-carboxamid; endothal; EPTC; esprocarb; ethalfluralin; ethametsulfuron-methyl; ethidimuron; ethiozin; ethofumesate; F5231, d. h. N-[2-Chlor-4-fluor-5-[4-(3-fluorpropyl)-4,5-dihydro-5-oxo-1H-tetrazol-1-yl]-phenyl]-ethansulfonamid; F6285, d. h. 1-[5-(N-Methylsulfonyl)-amino-2,4-dichlorophenyl]-3-methyl-4-difluoromethyl-1,2,4-triazol-5-on; fenoprop; fenoxan, s. clomazon; fenoxaprop-ethyl; fenuron; flamprop-methyl; flazasulfuron; fluazifop und dessen Esterderivate; fluchloralin; flumetsulam; N-[2,6-Difluorphenyl]-5-methyl-(1,2,4)-triazolo[1,5a]pyrimidin-2-sulfonamid; flumeturon; flumipropyn; fluorodifen; fluoroglycofen-ethyl; fluridone; flurochloridone; fluroxypyr; flurtamone; fomesafen; fosamine; furyloxyfen; glufosinate; glyphosate; halosaten; haloxyfop und dessen Esterderivate; hexazinone; Hw 52, d. h. N-(2,3-Dichlorphenyl)-4-(ethoxymethoxy)-benzamid; imazamethabenz-methyl; imazapyr; imazaquin; imazethamethapyr; imazethapyr; imazosulfuron; ioxynil; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; methabenzthiazuron; metham; methazole; methoxyphenone; methyldymron; metobromuron; metolachlor; metoxuron; metribuzin; metsulfuron-methyl; MH; molinate; monalide; monocarbamide dihydrogensulfate; monolinuron; monuron; MT 128, d. h. 6-Chlor-N-(3-chlor-2-propenyl)-5-methyl-N-phenyl-3-pyridazinamin; MT 5950, d. h. N-[3-Chlor-4-(1-methylethyl)-phenyl]-2-methylpentanamid; naproanilide; napropamide; naptalam; NC 310, d. h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiazon; oxyfluorfen; paraquat; pebulate; pendimethalin; perfluidone; phenmedipham; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Esterderivate; propazine; propham; propyzamide; prosulfalin; prosulfocarb; prynachlor; pyrazolinate; pyrazon; pyrazosulfuron-ethyl; pyrazoxyfen; pyridate; quinclorac; quinmerac; quinofop und dessen Esterderivate, quizalofop und dessen Esterderivate; quizalofop-ethyl; quizalofop-p-tefuryl; renriduron; dymron; S 275, d. h. 2-[4-Chlor-2-fluor-5-(2-propynyloxy)-phenyl]-4,5,6,7-tetrahydro-2H-indazol; S 482, d. h. 2-[7-Fluor-3,4-dihydro-3-oxo-4-(2-propynyl)-2H-1,4-benzoxazin-6-yl]-4,5,6,7-tetrahydro-1H-isoindol-1,3(2H)-dion; secbumeton; sethoxydim; siduron; simazine; simetryn; SN 106279, d. h. 2-[[7-[2-Chlor-4-(trifluor-methyl)-phenoxy]-2-naphthalenyl]-oxy]-propansäure und -methylester; sulfometuron-methyl; sulfazuron; flazasulfuron; TCA; tebutam; tebuthiuron; terbacil; terbucarb; terbuchlor; terbumeton; terbuthylazine; terbutryn; TFH 450, d. h. N,N-Diethyl-3-[(2-ethyl-6-methylphenyl)-sulfonyl]-1H-1,2,4-triazol-1-carboxamid; thiazafluron; thifensulfuron-methyl; thiobencarb; tiocarbazil; tralkoxydim; tri-allate; triasulfuron; triazofenamide; tribenuron-methyl; triclopyr; tridiphane; trietazine; trifluralin; trimeturon; vernolate; WL 110547, d. h. 5-Phenoxy-1-[3-(trifluormethyl)-phenyl]-1H-tetrazol.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser, und anschließend auf die Pflanzen, Pflanzenteile oder den landwirtschaftlich oder industriell genützten Boden, auf dem die Pflanzen stehen oder in dem sie heranwachsen oder als Saat vorliegen, appliziert. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

### A. Chemische Beispiele

### a) 2-Chlor-3-nitrobenzoesäuremethylester

25,50 g (0,127 Mol) 2-Chlor-3-nitrobenzoesäure werden in 50 ml MeOH gelöst und mit 3 ml konz. Schwefelsäure versetzt. Das Reaktionsgemisch wird 4 h zum Sieden erhitzt. Nach der Zugabe von 10 ml Orthoessigsäuretrimethylester wird das Gemisch für weitere 2 h zum Sieden erhitzt. Nach Abkühlen auf 0°C und Absaugen des Feststoffes erhält man 26,4 g (96 %) farblosen 2-Chlor-3-nitro-benzoesäuremethylester; Schmp. 68 - 70°C.

### b) 2-Benzylmercapto-3-nitro-benzoesäuremethylester

Zu einem Gemisch aus 50,0 g (0,23 Mol) 2-Chlor-3-nitro-benzoesäuremethylester und 28,81 g (0,23 Mol) Benzylmercaptan in 200 ml Methanol wird eine Lösung aus 26,0 g (0,23 Mol) Kalium-tert.-butylat in 400 ml MeOH zugetropft. Nach 6 h Rühren bei Raumtemperatur und weiteren 15 h Stehen wird das Reaktionsgemisch eingeengt. Der Rückstand wird in Ethylacetat aufgenommen und nacheinander mit ges. NaHCO₃-Lösung, Wasser und ges. NaCI-Lösung gewaschen. Nach dem Trocknen über MgSO₄ wird die Lösung unter reduziertem Druck eingeengt. Der gelbliche, ölige Rückstand (68,5 g, 97 % d. Th.) wird ohne weitere Reinigung für die nachfolgenden Umsetzungen eingesetzt (vgl. Beispiel c).

### c) 3-Amino-2-benzylmercapto-benzoesäuremethylester

Zu einem Gemisch aus 68,0 g (0,22 Mol) 2-Benzylmercapto-3-nitro-benzoesäuremethylester, 215 ml Eisessig und 480 ml Wasser werden portionsweise 69,8 g Eisenpulver zugesetzt. Anschließend wird die Reaktionslösung 3 h bei 50 - 60°C gerührt. Nach dem Abtrennen des Feststoffes durch Filtration wird die Mutterlauge mit Wasser und Salz gewaschen und anschließend über MgSO₄ getrocknet. Nach Einengen der Lösung unter reduziertem Druck erhält man 47,1 g eines gelben Öls (77 %); ¹H-NMR (CDCl₃, 80 MHz): δ = 3.8 (s, 3H, OCH₃), 3.9 (s, 2H, SCH₂Ph), 5.9 (s, 2H, NH₂), 6.6-7.3 (m, 8H, H_{arom.}).

### d) 2-Benzylmercapto-3-formylamino-benzoesäuremethylester

Bei 0°C werden zu 48,6 g (0,476 Mol) Essigsäureanhydrid 27,0 g (0,586 Mol) Ameisensäure zugetropft. Nach 2 h Rühren bei 50 - 60°C wird das Gemisch auf 0°C abgekühlt und mit einer Lösung aus 50,0 g (0,183 Mol) 3-Amino-2-benzylmercapto-benzoesäuremethylester in 150 ml THF versetzt. Anschließend wird die Reaktionslösung 4 h bei Raumtemperatur gerührt. Das Gemisch wird unter reduziertem Druck (60°C, 0.1 Torr) eingeengt. Der erhaltene, ölige Rückstand (61,77 g) wird ohne weitere Reinigung in nachfolgende Reaktionen eingesetzt (vgl. Beispiel e)).

### e) 2-Benzylmercapto-3-methylamino-benzoesäuremethylester

Zu einer Lösung aus 61,5 g 2-Benzylmercapto-3-formyl-amino-benzoesäuremethylester (vgl. Beispiel d)) in 100 ml CHCl₃ werden bei 0°C 20 ml Borandimethylsulfid-komplex (BMS) zugetropft. Man erwärmt anschließend für 2 h auf 60°C, setzt weitere 20 ml BMS hinzu und rührt das Gemisch weitere 2,5 h bei dieser Temperatur. Anschließend werden bei 0°C 30 ml Methanol zugetropft. Das Gemisch wird in einen Scheidetrichter überführt, mit Wasser und Sole gewaschen und anschließend über MgSO₄ getrocknet. Nach dem Abdestillieren flüchtiger Komponenten werden 54,60 g 2-Benzylmercapto-3-methylamino-benzoesäuremethylester in Form eines gelblichen Öls erhalten. ¹H-NMR (CDCl₃, 80 MHz): δ = 2.6 (d, 3H, NH-CH₃), 3.8 (s, 3H, OCH₃), 3.9 (s, 2H, SCH₂Ph), 5.2 (s, breit, 1H, NH-Me), 6.5 (d, 1H, H_{arom.}), 6.8 (d, 1H, H_{arom.}), 7.0-7.4 (m, 6H, H_{arom.}).

### f) 2-Benzylmercapto-3-(N-methoxycarbonyl-methylamino)-benzoesäuremethylester

Zu einer Suspension aus 15,0 g (0,052 Mol) 2-Benzylmercapto-3-methylamino-benzoesäuremethylester und 5,88 g (0,07 Mol) Natriumhydrogencarbonat in 100 ml CH₂CN werden 4,6 ml Chlorameisen-säuremethylester zugetropft. Nach 5 h werden flüchtige Komponenten abdestilliert (15 Torr, 40°C). Der Rückstand wird in Ethylacetat aufgenommen und nacheinander mit 1 N Salzsäure und Wasser gewaschen. Nach dem Trocknen der organischen Phase über MgSO₄ wird das Solvens abdestilliert. 2-Benzylmercapto-3-(N-methoxycarbonyl-methylamino)-benzoesäuremethylester fällt in Form eines gelblichen Öls (14,8 g ) an; ¹H-NMR (CDCl₃, 80 MHz): δ = 3.0 (s, 3H, N-CH₃), 3.6 (s, 3H, O-CH₃) 3.9 (s, 5H, OCH₃ und SCH₂Ph), 7.1-7.6 (m, 8H, H_{arom.}).

### g) 2-Chlorsulfonyl-3-(N-methoxycarbonyl-methylamino)-benzoesäuremethylester

In ein Gemisch aus 8,10 g 2-Benzylmercapto-3-(N-methoxycarbonyl-methylamino)-benzoesäuremethylester in 50 ml CH₂Cl₂ und 150 ml Kaliumdihydrogenphosphat-Dinatriumhydrogenphosphat-Lösung (Riedel-de Haën AG, pH = 7) wird bei einer Temperatur zwischen 0°C und 5°C Chlor eingeleitet. Nach 30 min wird Argon durch die Reaktionsapparatur durchgeleitet, um überschüssiges Chlor zu entfernen. Nach der Phasentrennung wird die organische Phase über MgSO₄ getrocknet und anschließend unter reduziertem Druck eingeengt. Durch Kristallisation aus Ethylacetat/Hexan werden 4,5 g an 2-Chlorsulfonyl-3-(N-methoxycarbonyl-methylamino)-benzoesäuremethylester erhalten; Schmp.: 102-105°C.

### h) N-[(4-Dimethylamino-6-trifluorethoxy-triazin-2-yl)-aminocarbonyl]-2-methoxycarbonyl-6-(N-methoxycarbonyl-methylamino)-benzolsulfonamid (Tabelle 1, Beispiel 24)

Zu einer Suspension aus 0,25 g (0,0038 Mol) NaOCN, 0,6 ml Pyridin und 12 ml Acetonitril werden bei Raumtemperatur nacheinander 0,71 g (0,003 Mol) 2-Amino-4-dimethylamino-6-(2,2,2-trifluorethoxy)-triazin und 1,0 g (0,003 Mol) 2-Chlorsulfonyl-3-(N-methoxycarbonyl-methylamino)-benzoesäuremethylester zugesetzt. Nach 3.5 h Rühren bei Raumtemperatur wird das Reaktionsgemisch auf Eiswasser gegossen. Zur Reinigung wird der abgeschiedene Sulfonylharnstoff in wenig Methanol gerührt, mittels Filtration abgetrennt und getrocknet. Der Sulfonylharnstoff fällt als farbloser Feststoff an (0,75 g). Schmp.: 125-126 °C.

### i) N-[(4,6-Dimethoxypyrimidin-2-yl)-aminocarbonyl]-2-methoxycarbonyl-6-(N-methoxycarbonyl-methylamino)-benzolsulfonamid (Tabelle 1, Beispiel 13)

Zu einer Suspension aus 0,61 g Natriumcyanat, 0,8 ml Pyridin und 18 ml Acetonitril werden bei Raumtemperatur nacheinander 0,54 g 2-Amino-4,6-dimethoxypyrimidin und 1,50 g 2-Chlorsulfonyl-3-(N-methoxycarbonyl-methylamino)-benzoesäuremethylester zugesetzt. Nach 3,5 h Rühren bei Raumtemperatur wird das Reaktionsgemisch auf Eiswasser gegossen. Zur Reinigung wird der abgeschiedene Sulfonylharnstoff in wenig Methanol gerührt, mittels Filtration abgetrennt und getrocknet. Das so erhaltene N-[(4,6-Dimethoxypyrimidin-2-yl)-aminocarbonyl]-2-methoxycarbonyl-6-(N-methoxycarbonyl-methylamino)-benzolsulfonamid fällt als farbloser Feststoff (1,35 g) an; 150-154 °C.

### j) N-[(4,6-Dimethoxypyrimidin-2-yl)-aminocarbonyl]-2-methoxycarbonyl-4-(N-methoxycarbonyl-methylamino)-benzolsulfonamid (Tabelle 2, Beispiel 80)

Zu einem Gemisch aus 0,87 g 2-Methoxycarbonyl-4-(N-methoxycarbonyl-methylamino)-benzolsulfonamid und 0,90 g 4,6-Dimethoxy-2-phenoxycarbonylamino-pyrimidin in 10 ml Acetonitril werden bei 0°C 0,6 ml DBU zugetropft. Anschließend wird die Reaktionslösung 6 h bei Raumtemperatur gerührt. Nach dem Abdestillieren des Solvens wird der Rückstand in Wasser aufgenommen und mit Diethylether gewaschen. Nach dem Ansäuern der wäßrigen Phase mit konz. Salzsäure (pH = 1) wird der abgeschiedene Sulfonylharnstoff in wenig Methanol gerührt. Nach Abtrennen des farblosen Feststoffes mittels Filtration und Trocknen werden 1,18 g an N-[(4,6-Dimethoxypyrimidin-2-yl)-aminocarbonyl]-2-methoxycarbonyl-4-(N-methoxycarbonyl-methylamino)-benzolsulfonamid erhalten; Schmp.: 184-186°C (Zers.). Das vorstehende Verfahren wird mit folgenden Einsatzmengen wiederholt: 31,8 g eingesetztes Benzolsulfonamid, 58,0 g eingesetztes Pyrimidin, 150 ml Acetonitril, 39,4 ml DBU. Nach 4 h Umsetzung bei Raumtemperatur und Aufarbeitung wie oben beschrieben werden 45.0 g Produkt mit einem Schmp. von 187-189° C erhalten.

### k) N-[(4,6-Dimethoxy-pyrimidin-2-yl)-aminocarbonyl]-2-methoxycarbonyl-4-(N-methoxycarbonyl-methylamino)-benzolsulfonamid-Natriumsalz (Tabelle 4, Beispiel 1)

0,53 g N-[(4,6-Dimethylpyrimidin-2-yl)-aminocarbonyl]-2-methoxycarbonyl-4-(N-methoxycarbonyl-methylamino)-benzolsulfonamid werden in 50 ml CH₂Cl₂ und 30 ml CH₃CN bei Raumtemperatur gerührt und mit 1,1 ml 1N Natronlauge versetzt. Nach 2 h Rühren wird die Reaktionslösung unter reduziertem Druck eingeengt (50°C, 0,1 Torr). Das farblose Salz (0,59 g) fällt als farbloser Feststoff an; Schmp.: 155-158°C;
bei Einsatz von 15,0 g des substituierten Benzolsulfonamids, 120 ml CH3CN als alleinigem Lösungsmittel, 30 ml 1 N Natronlauge werden entsprechend 16,2 g Produkt mit einem Schmelzpunkt von 166° C (Zers.) erhalten.

Die in den nachfolgenden Tabelle 1 und 3 beschriebenen Verbindungen werden nach bzw. analog den obigen Beispielen h) bis k) erhalten.

Abkürzungen in den Tabellen 1-3:
- Tab.Bsp =: Tabelle, Beispiel Nr.
- Fp. =: Schmelzpunkt (Festpunkt)
- Et =: Ethyl
- Me =: Methyl
- Pr =: ⁿPr = n-Propyl
- ⁱPr =: Isopropyl
- ^{c}Pr =: Cyclopropyl
- (Z) =: (Zers.) = Zersetzungspunkt, Fp. unter Zersetzung
- CH₂CCH =: Propargyl

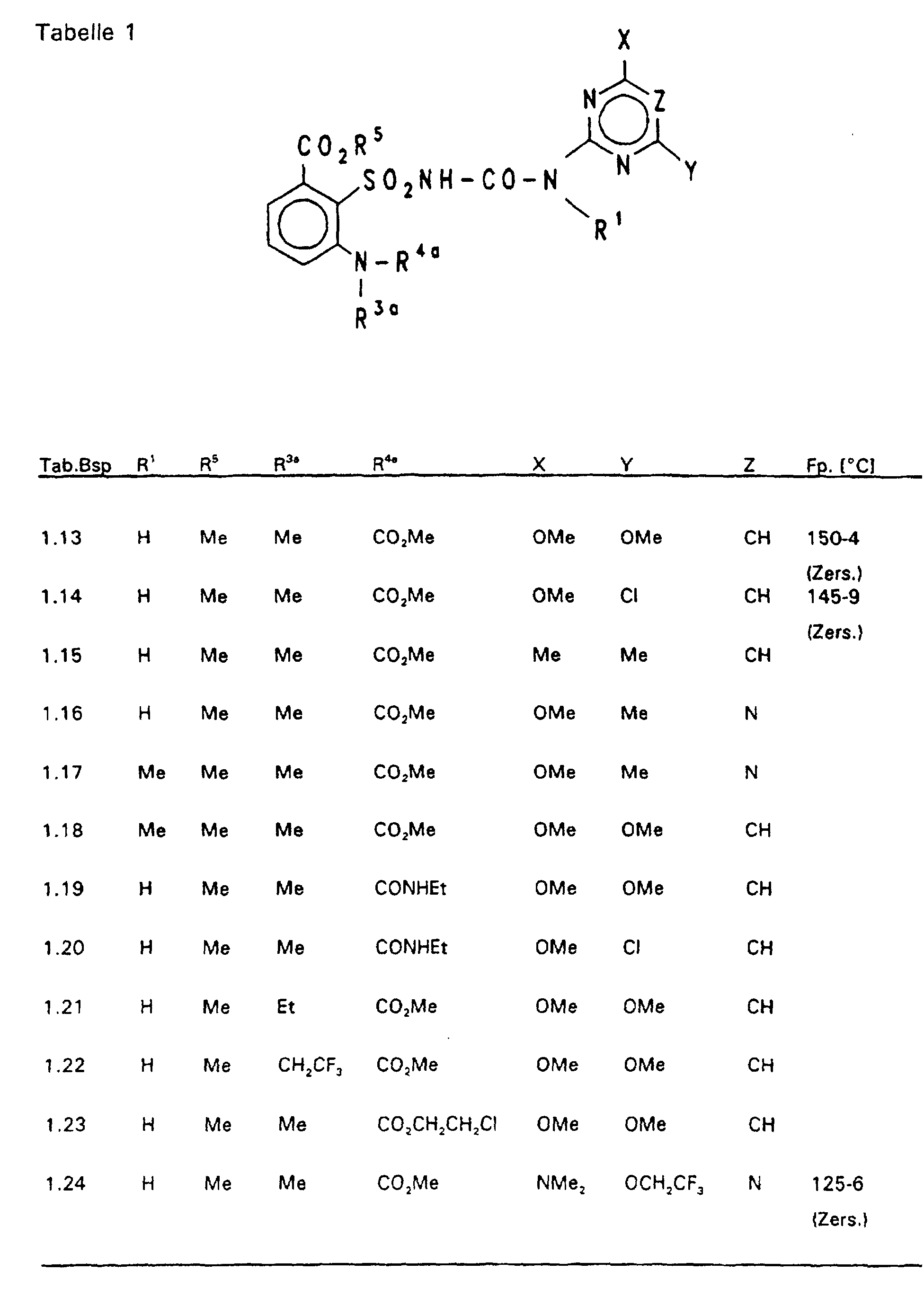

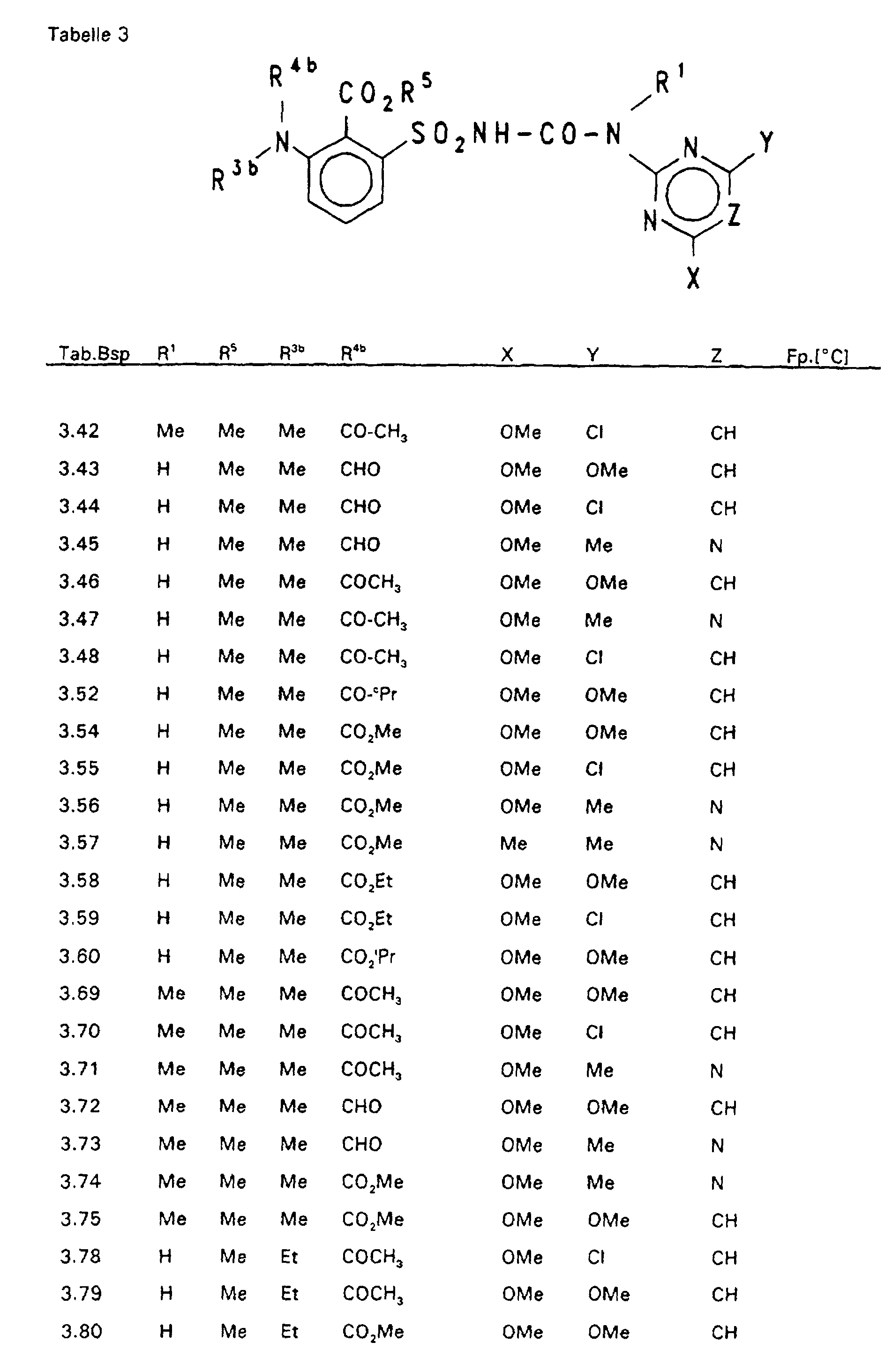

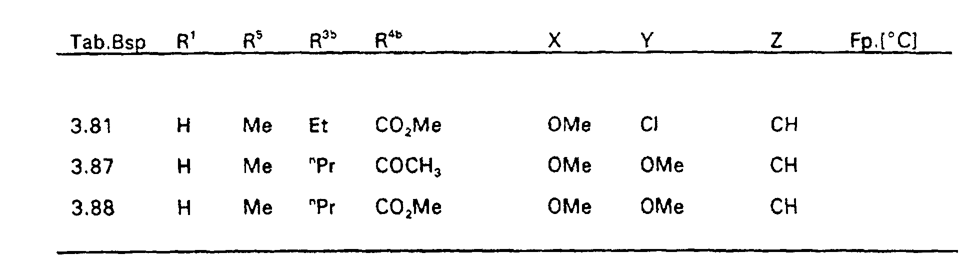

### B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gewichtsteilen Alkylphenolpolyglykolether (Triton X 207), 3 Gewichtsteilen Isotridecanolpolyglykolether (8 EO) und 71 Gewichtsteilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gewichtsteilen einer Verbindung der Formel (I), 75 Gewichtsteilen Cyclohexanon als Lösungsmittel und 10 Gewichtsteilen oxethyliertes Nonylphenol als Emulgator.
e) Ein in Wasser dispergierbares Granulat wird erhalten indem man 75 Gewichtsteile einer Verbindung der Formel (I),
   10 Gewichtsteile ligninsulfonsaures Calcium,
   5 Gewichtsteile Natriumlaurylsulfat,
   3 Gewichtsteile Polyvinylalkohol und
   7 Gewichtsteile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gewichtsteile einer Verbindung der Formel (I),
   5 Gewichtsteile 2,2'-dinaphthylmethan-6,6'-disulfonsaures Natrium
   2 Gewichtsteile oleoylmethyltaurinsaures Natrium,
   1 Gewichtsteil Polyvinylalkohol,
   17 Gewichtsteile Calciumcarbonat und
   50 Gewichtsteile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Unkrautwirkung im Vorauflauf

Samen bzw. Rhizomstücke von mono- und dikotylen Unkraufpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspension bzw. Emulsion mit einer Wasseraufwandmenge von umgerechnet 600 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 3 bis 4 Wochen im Vergleich zu unbehandelten Kontrollen. Wie die Testergebnisse zeigen, weisen die erfindungsgemäßen Verbindungen eine gute herbizide Vorauflaufwirksamkeit gegen ein breites Spektrum von Ungräsern und Unkräutern auf. Beispielsweise haben die Verbindungen der Beispiele 1.13, 1.14, 1.24, 2.1, 2.15, 2.69, 2.80, 2.81, 2.92, 2.101, 2.105, 2.106, 2.119-2.125, 4.1, 4.3, 4.6, 4,8, 4.14, 4.15 und 4.16 aus den Tabellen 1-4 sehr gute herbizide Wirkung gegen Schadpflanzen wie Sinapis alba, Chrysanthemum segetum, Avena sativa, Stellaria media, Echinochloa crus-galli und Lolium multiflorum im Vorauflaufverfahren bei einer Aufwandmenge von 0,3 kg und weniger Aktivsubstanz pro Hektar.

### 2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise haben die Verbindungen der Beispiele 1.13, 1.14, 1.24, 2.1, 2.15, 2.69, 2.80, 2.81, 2.92, 2.101, 2.105, 2.106, 2.119-2.125, 4.1, 4.3, 4.6, 4.8, 4.14, 4.15 und 4.16 aus den Tabellen 1-4 sehr gute herbizide Wirkung gegen Schadpflanzen wie Sinapis alba, Stellaria media, Echinochloa crus-galli, Lolium multiflorum, Chrysanthemum segetum und Avena sativa im Nachauflaufverfahren bei einer Aufwandmenge von 0,3 kg und weniger Aktivsubstanz pro Hektar.

### 3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt. Ein Teil der Töpfe wurde sofort wie unter Abschnitt 1 beschrieben behandelt, die übrigen im Gewächshaus aufgestellt, bis die Pflanzen zwei bis drei echte Blätter entwickelt hatten und dann wie unter Abschnitt 2 beschrieben mit den erfindungsgemäßen Substanzen der Formel (I) in unterschiedlichen Dosierungen besprüht. Vier bis fünf Wochen nach der Applikation und Standzeit im Gewächshaus wurde mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus aus Gramineen-Kulturen wie z.B. Gerste, Weizen, Roggen, Sorghum-Hirsen, Mais oder Reis. Die Verbindungen der Formel (I) weisen somit eine hohe Selektivität bei Anwendung zur Bekämpfung von unerwünschten Pflanzenwuchs in landwirtschaftlichen Kulturen auf.

## Patentansprüche

1. Verbindungen der Formel (I) und deren Salze, 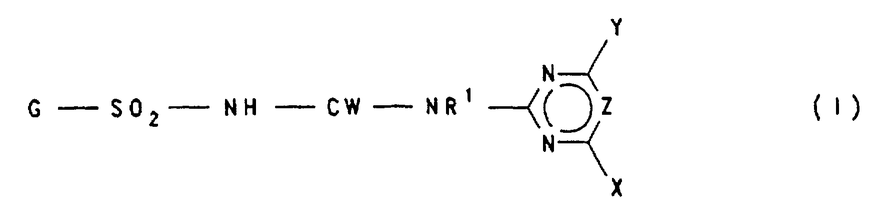 worin
G einen substituierten N-Acyl-aminophenylrest aus der Gruppe G¹ und G³, 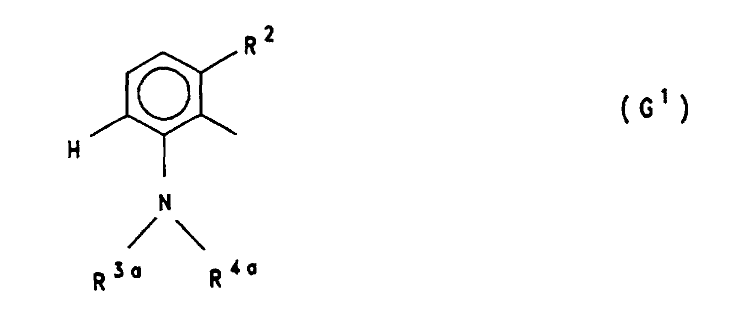 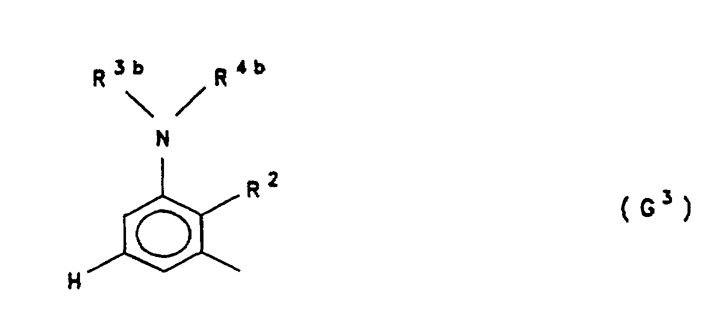
R¹ H oder C₁-C₃-Alkyl,
R² CO-OR⁵, CS-SR⁶, CO-SR⁷, CS-OR⁸, CO-R⁹, CO-NR¹⁰R¹¹, CO-O-N=CR¹²R¹³, C(=NR¹⁴)R¹⁵ oder CS-NR¹⁶R¹⁷,
R^{3a} C₁-C₅-Alkyl, C₂-C₅-Alkenyl oder C₂-C₅-Alkinyl, wobei die drei letztgenannten Reste unsubstituiert oder durch einen oder mehrere Reste aus der Gruppe Halogen, C₁-C₄-Alkoxy, CN, Di-(C₁-C₄-Alkyl)-amino, N₃, und C₁-C₃-Alkylthio substituiert sind,
R^{3b} C₁-C₅-Alkyl, C₃-C₈-Cycloalkyl, C₂-C₅-Alkenyl oder C₂-C₅-Alkinyl, wobei die vier letztgenannten Reste unsubstituiert oder durch einen oder mehrere Reste aus der Gruppe Halogen, C₁-C₄-Alkoxy, CN, Di-(C₁-C₄-Alkyl)-amino, N₃, und C₁-C₃-Alkylthio substituiert sind,
R^{4a} CO-(C₁-C₅-Alkyl) oder CO₂-(C₁-C₅-Alkyl),
wobei die zwei letztgenannten Reste unsubstituiert oder durch einen oder mehrere Reste aus der Gruppe OH, NR¹⁸R¹⁹, N₃, CN, CO₂H, S(O)ₓ-(C₁-C₄-Alkyl) und CO₂-(C₁-C₃-Alkyl) substituiert sind,
oder Aminocarbonyl oder Aminosulfonyl, wobei die beiden letztgenannten Reste unsubstituiert oder durch gleiche oder verschiedene Reste aus der Gruppe C₁-C₄-Alkyl und C₁-C₄-Haloalkyl N-substituiert sind,
R^{4b} CO-(C₁-C₅-Alkyl), CO-(C₃-C₆-Cycloalkyl), CO₂-(C₁-C₅-Alkyl), CS-(C₁-C₅-Alkyl), CO-S-(C₁-C₆-Alkyl), CS-O-(C₁-C₆-Alkyl) oder CS-S-(C₁-C₆-Alkyl), wobei die sieben letztgenannten Reste unsubstituiert oder durch einen oder mehrere Reste aus der Gruppe, CN, N₃, CO₂H, CO₂-(C₁-C₃-Alkyl), OH, NR²⁰R²¹, S(O)ₓ-(C₁-C₄-Alkyl)und CO-NR²²R²³ substituiert sind,
oder SO₂NR²⁹R³⁰,
R⁵ C₁-C₅-Alkyl, C₂-C₆-Alkenyl, C₂-C₅-Alkinyl, C₃-C₆-Cycloalkyl oder C₄-C₇-Cycloalkylalkyl, wobei die fünf letztgenannten Reste unsubstituiert oder durch einen oder mehrere Reste aus der Gruppe Halogen, CN, N₃, C₁-C₃-Alkoxy, C₁-C₃-Alkylthio, Amino, Mono- und Di-(C₁-C₄-alkyl)-amino, NO₂, SCN, C₁-C₃-Haloalkoxy und C₁-C₃-Haloalkylthio substituiert sind,
R⁸ einen Rest wie R⁵.
R⁷ einen Rest wie R⁵,
R⁸ einen Rest wie R⁵,
R⁹ H, C₁-C₆-Alkyl, C₂-C₆-Alkenyl, C₂-C₆-Alkinyl, C₃-C₇-Cycloalkyl, wobei die vier letztgenannten Reste unabhängig voneinander unsubstituiert oder durch einen oder mehrere Reste aus der Gruppe Halogen, C₁-C₄-Alkoxy, C₁-C₄-Alkylthio, C₁-C₄-Haloalkoxy, C₁-C₄-Haloalkylthio und CN substituiert sind,
R¹⁰ H, C₁-C₆-Alkyl, C₂-C₆-Alkenyl, C₂-C₆-Alkinyl, wobei die drei letztgenannten Reste unsubstituiert oder durch einen oder mehrere Reste aus der Gruppe C₁-C₃-Alkoxy, C₁-C₃-Alkylthio, Halogen, CN, Amino, C₁-C₄-Alkyl-amino und Di-(C₁-C₄-alkyl)-amino substituiert sind, oder C₁-C₄-Alkoxy oder OH,
R¹¹ H, C₁-C₆-Alkyl, C₂-C₆-Alkenyl, C₂-C₆-Alkinyl, wobei die drei letztgenannten Reste unsubstituiert oder durch einen oder mehrere Reste aus der Gruppe Halogen, CN, NH₂, Mono- und Di-(C₁-C₄-alkyl)-amino, C₁-C₃-Alkoxy und C₁-C₃-Alkylthio substituiert sind, oder
NR¹⁰R¹¹ gemeinsam einen heterocyclischen Ring, der neben dem N-Ringatom noch bis zu zwei Heteroringatome aus der Gruppe N, O und S enthalten und weiter substituiert sein kann,
R¹² und R¹³ unabhängig voneinander H, C₁-C₄-Alkyl, C₂-C₄-Alkenyl oder C₂-C₄-Alkinyl oder
R¹² und R¹³ gemeinsam eine Alkylenkette mit 3 oder 4 C-Atomen, die noch mit C₁-C₄-Alkyl oder C₁-C₄-Alkoxy substituiert sein kann,
R¹⁴ H, OH, NH₂, NHR³¹, NR³¹₂, C₁-C₄-Alkyl, C₁-C₄-Alkoxy, C₂-C₄-Alkenyl oder C₂-C4-Alkinyl, wobei die letztgenannten vier Reste unsubstituiert oder durch einen oder mehrere Reste aus der Gruppe Halogen, C₁-C₃-Alkoxy und C₁-C₃-Alkylthio substituiert sind,
R¹⁵ H, C₁-C₆-Alkyl, C₁-C₆-Alkoxy, C₂-C₆-Alkenyl, C₂-C₆-Alkinyl, wobei die vier letztgenannten Reste unsubstituiert oder durch einen oder mehrere Reste aus der Gruppe Halogen, C₁-C₃-Alkoxy, C₁-C₃-Alkylthio substituiert sind,
R¹⁶ einen Rest wie R¹⁰,
R¹⁷ einen Rest wie R¹¹,
R¹⁸ unabhängig von R¹⁹ H, C₁-C₄-Alkyl, C₂-C₅-Alkenyl oder C₂-C₅-Alkinyl, wobei die drei letztgenannten Reste unsubstituiert oder durch einen oder mehrere Reste aus der Gruppe Halogen, C₁-C₃-Alkoxy, C₁-C₃-Alkylthio und CN substituiert sind,
R¹⁹ unabhängig von R¹⁸ einen Rest wie R¹⁸ oder C₁-C₃-Alkoxy oder OH oder
NR¹⁸R¹⁹ gemeinsam einen heterocyclischen Ring, der neben dem N-Ringatom noch bis zu zwei Heteroringatome aus der Gruppe N, O und S enthalten und weiter substituiert sein kann,
R²⁰ unabhängig von R²¹ H, C₁-C₄-Alkyl, C₂-C₄-Alkenyl oder C₂-C₄-Alkinyl, wobei die drei letztgenannten Reste unsubstituiert oder durch einen oder mehrere Reste aus der Gruppe Halogen, CN, NH₂, Mono- und Di-(C₁-C₄)-alkylamino, C₁-C₃-Alkoxy, C₁-C₃-Alkylthio und CO₂-(C₁-C₃-Alkyl) substituiert sind,
R²¹ unabhängig von R²⁰ einen Rest wie R²⁰ oder CHO, CO-(C₁-C₅-Alkyl) oder CO₂-(C₁-C₅-Alkyl) oder
NR²⁰R²¹ einen heterocyclischen Ring analog NR¹⁸R¹⁹,
R²² und R²³ unabhängig voneinander H, C₁-C₃-Alkyl, C₂-C₄-Alkenyl oder C₂-C₄-Alkinyl oder
NR²²R²³ einen heterocyclischen Ring analog NR¹⁸R¹⁹,
R²⁹ unabhängig von R³⁰ H, C₁-C₄-Alkyl, C₂-C₅-Alkenyl oder C₂-C₅-Alkinyl, wobei die drei letztgenannten Reste unsubstituiert oder durch einen oder mehrere Reste aus der Gruppe Halogen, C₁-C₃-Alkoxy, C₁-C₃-Alkylthio und CN substituiert sind,
R³⁰ unabhängig von R²⁹ H, C₁-C₄-Alkyl, C₂-C₅-Alkenyl oder C₂-C₅-Alkinyl, wobei die drei letztgenannten Reste unsubstituiert oder durch einen oder mehrere Reste aus der Gruppe Halogen, C₁-C₃-Alkoxy, C₁-C₃-Alkylthio und CN substituiert sind, oder C₁-C₃-Alkoxy oder OH oder
NR²⁹R³⁰ einen heterocyclischen Ring analog NR¹⁸R¹⁹,
R³¹ C₁-C₄-Alkyl, C₂-C₄-Alkenyl oder C₂-C₄-Alkinyl, wobei die drei letztgenannten Reste unabhängig voneinander unsubstituiert oder durch einen oder mehrere Reste aus der Gruppe Halogen, C₁-C₄-Alkoxy und C₁-C₄-Alkylthio substituiert sind,
x - unabhängig von den übrigen Indices x - 0, 1 oder 2,
W O oder S,
X, Y unabhängig voneinander Wasserstoff, Halogen, C₁-C₂-Alkyl, C₁-C₂-Alkoxy oder C₁-C₂-Alkylthio, wobei die drei letztgenannten Reste unsubstituiert oder durch einen oder mehrere Reste aus der Gruppe Halogen, C₁-C₂-Alkoxy und C₁-C₂-Alkylthio substituiert sind, oder Mono- oder Di(C₁-C₂-alkyl)amino, C₃-C₄-Alkenyl, C₃-C₄-Alkenyloxy oder C₃-C₄-Alkinyloxy und
Z CH oder N
bedeuten.

2. Verbindungen und deren Salze nach Anspruch 1, **dadurch gekennzeichnet, daß**
G ein Rest der Formel (G1) ist und
R¹ H oder C₁-C₃-Alkyl,
R² CO-OR⁵, CS-SR⁶, CO-SR⁷, CS-OR⁸, CO-R⁹, CO-NR¹⁰R¹¹, CO-O-N=CR¹²R¹³, C(=NR¹⁴)R¹⁵ oder CS-NR¹⁶R¹⁷,
R^{3a} C₁-C₄-Alkyl, C₁-C₄-Haloalkyl, C₂-C₄-Alkenyl oder C₂-C₄-Alkinyl,
R^{4a} CO-(C₁-C₄-Alkyl), CO₂-(C₁-C₅-Alkyl), CO-NH₂, CO-NH(C₁-C₄-Alkyl), CO-N(C₁-C₄-Alkyl)₂ oder SO₂-NH₂, SO₂-NH(C₁-C₄-Alkyl) oder SO₂-N(C₁-C₄-Alkyl)₂,
R⁵ C₁-C₄-Alkyl, C₁-C₄-Haloalkyl, C₂-C₄-Alkenyl, C₂-C₄-Alkinyl, C₃-C₆-Cycloalkyl oder C₄-C₇-Cycloalkylmethyl,
R⁶ einen Rest wie R⁵,
R⁷ einen Rest wie R⁵,
R⁸ einen Rest wie R⁵,
R⁹ H, C₁-C₄-Alkyl, C₂-C₄-Alkenyl, C₂-C₄-Alkinyl oder C₃-C₆-Cycloalkyl, wobei die vier letztgenannten Reste unabhängig voneinander unsubstituiert oder durch einen oder mehrere Reste aus der Gruppe Halogen, C₁-C₂-Alkoxy, C₁-C₂-Alkylthio, C₁-C₂-Haloalkoxy, C₁-C₂-Haloalkylthio und CN substituiert sind,
R¹⁰ und R" unabhängig voneinander H, C₁-C₄-Alkyl, C₂-C₄-Alkenyl oder C₂-C₄-Alkinyl,
R¹² und R¹³ unabhängig voneinander H, C₁-C₄-Alkyl, C₂-C₄-Alkenyl oder C₂-C₄-Alkinyl oder
R¹² und R¹³ gemeinsam eine Alkylenkette mit 3 oder 4 C-Atomen, die noch mit C₁-C₄-Alkyl oder C₁-C₄-Alkoxy substituiert sein kann,
R¹⁴ NH₂, NHR³¹, NR³¹₂, C₁-C₄-Alkyl, C₁-C₄-Alkoxy, C₂-C₄-Alkenyl oder C₂-C₄-Alkinyl,
R¹⁵ H, C₁-C₄-Alkyl, C₁-C₄-Alkoxy, C₂-C₄-Alkenyl oder C₂-C₄-Alkinyl,
R¹⁶ einen Rest wie R¹⁰,
R¹⁷ einen Rest wie R¹¹,
R³¹ C₁-C₄-Alkyl, C₂-C₄-Alkenyl oder C₂-C₄-Alkinyl,
W O oder S,
X, Y unabhängig voneinander Halogen, C₁-C₂-Alkyl, C₁-C₂-Haloalkyl, C₁-C₂-Alkoxy, C₁-C₂-Haloalkoxy, C₁-C₂-Alkylthio, Mono- oder Di(C₁-C₂-alkyl)amino, C₃-C₄-Alkenyl, C₃-C₄-Alkenyloxy oder C₃-C₄-Alkinyloxy, und
Z CH oder N
bedeuten.

3. Verbindungen und deren Salze nach Anspruch 1 oder 2, **dadurch gekennzeichnet, daß**
G ein Rest der Formel (G3) ist und
R¹ H oder C₁-C₃-Alkyl,
R² CO-OR⁵, CS-SR⁶, CO-SR⁷, CS-OR⁸, CO-R⁹, CO-NR¹⁰R¹¹, CO-O-N=CR¹²R¹³, C(=NR¹⁴)R¹⁵ oder CS-NR¹⁶R¹⁷,
R^{3b} C₁-C₄-Alkyl, C₃-C₆-Cycloalkyl, C₁-C₄-Haloalkyl, C₂-C₄-Alkenyl oder C₂-C₄-Alkinyl,
R^{4b} CO-(C₁-C₄-Alkyl), CO-(C₃-C₆-Cycloalkyl) oder CO₂-(C₁-C₄-Alkyl), wobei die 3 letztgenannten Reste unsubstituiert oder durch einen oder mehrere Reste aus der Gruppe, CN, NR²⁰R²¹, S(O)ₓ-(C₁-C₄-Alkyl) und CO-NR²²R²³ sind,
oder SO₂NR²⁹R³⁰,
R⁵ C₁-C₄-Alkyl, C₂-C₄-Alkenyl, C₂-C₄-Alkinyl, C₃-C₆-Cycloalkyl oder C₄-C₇-Cycloalkylmethyl, wobei die fünf letztgenannten Reste unsubstituiert oder durch einen oder mehrere Reste aus der Gruppe Halogen, C₁-C₂-Alkoxy und C₁-C₂-Haloalkoxy substituiert sind,
R⁶ einen Rest wie R⁵,
R⁷ einen Rest wie R⁵,
R⁸ einen Rest wie R⁵,
R⁹ H, C₁-C₄-Alkyl, C₂-C₄-Alkenyl, C₂-C₄-Alkinyl oder C₃-C₆-Cycloalkyl, wobei die vier letztgenannten Reste unabhängig voneinander unsubstituiert oder durch einen oder mehrere Reste aus der Gruppe Halogen, C₁-C₂-Alkoxy, C₁-C₂-Alkylthio, C₁-C₂-Haloalkoxy, C₁-C₂-Haloalkylthio und CN substituiert sind,
R¹⁰ und R¹¹ unabhängig voneinander H, C₁-C₄-Alkyl, C₂-C₄-Alkenyl oder C₂-C₄-Alkinyl,
R¹² und R¹³ unabhängig voneinander H, C₁-C₄-Alkyl, C₂-C₄-Alkenyl oder C₂-C₄-Alkinyl oder
R¹² und R¹³ gemeinsam eine Alkylenkette mit 3 oder 4 C-Atomen, die noch mit C₁-C₄-Alkyl oder C₁-C₄-Alkoxy substituiert sein kann,
R¹⁴ NH₂, NHR³¹, NR³¹₂, C₁-C₄-Alkyl, C₁-C₄-Alkoxy, C₂-C₄-Alkenyl oder C₂-C₄-Alkinyl,
R¹⁵ H, C₁-C₄-Alkyl, C₁-C₄-Alkoxy, C₂-C₄-Alkenyl oder C₂-C₄-Alkinyl,
R¹⁶ einen Rest wie R¹⁰,
R¹⁷ einen Rest wie R¹¹,
R²⁰ unabhängig von R²¹ H, C₁-C₄-Alkyl, C₂-C₄-Alkenyl oder C₂-C₄-Alkinyl, wobei die drei letztgenannten Reste unsubstituiert oder durch einen oder mehrere Reste aus der Gruppe Halogen und C₁-C₂-Alkoxy substituiert sind,
R²¹ unabhängig von R²⁰ einen Rest wie R²⁰ oder CHO, CO-(C₁-C₄-Alkyl) oder CO₂-(C₁-C₄-Alkyl) oder
NR²⁰R²¹ gemeinsam einen heterocyclischen Ring, der neben dem N-Ringatom noch ein Heteroringatom aus der Gruppe N, O und S enthalten und weiter durch Reste aus der Gruppe C₁-C₄-Alkyl, C₁-C₄-Alkoxy und Halogen substituiert und, im Falle von S als Heteroringatom, am S-Atom oxidiert sein kann,
R²² und R²³ unabhängig voneinander H, C₁-C₃-Alkyl, C₂-C₄-Alkenyl oder C₂-C₄-Alkinyl oder
NR²²R²³ einen heterocyclischen Ring analog NR²⁰R²¹,
R²⁹ unabhängig von R³⁰ H, C₁-C₄-Alkyl, C₂-C₄-Alkenyl, C₂-C₄-Alkinyl, wobei die drei letztgenannten Reste unsubstituiert oder durch einen oder mehrere Reste aus der Gruppe Halogen, C₁-C₃-Alkoxy, C₁-C₃-Alkylthio und CN substituiert sind,
R³⁰ unabhängig von R²⁹ H, C₁-C₄-Alkyl, C₂-C₄-Alkenyl, C₂-C₄-Alkinyl, wobei die drei letztgenannten Reste unsubstituiert oder durch einen oder mehrere Reste aus der Gruppe Halogen, C₁-C₃-Alkoxy, C₁-C₃-Alkylthio und CN substituiert sind, oder C₁-C₃-Alkoxy oder OH oder
NR²⁹R³⁰ einen heterocyclischen Ring analog NR²⁰R²¹,
R³¹ C₁-C₄-Alkyl, C₂-C₄-Alkenyl oder C₂-C₄-Alkinyl,
x 0, 1 oder 2,
W O oder S,
X, Y unabhängig voneinander Halogen, C₁-C₂-Alkyl, C₁-C₂-Haloalkyl, C₁-C₂-Alkoxy, C₁-C₂-Haloalkoxy, C₁-C₂-Alkylthio, Mono- oder Di(C₁-C₂-alkyl)amino, C₃-C₄-Alkenyl, C₃-C₄-Alkenyloxy oder C₃-C₄-Alkinyloxy, und
z CH oder N
bedeuten.

4. Verbindungen und deren Salze nach einem der Ansprüche 1 bis 3, **dadurch gekennzeichnet, daß** R² einen Rest der Formel CO-OR⁵ bedeutet.

5. Verfahren zur Herstellung von Verbindungen der Formel (I) oder deren Salzen, definiert nach einem der Ansprüche 1 bis 4, **dadurch gekennzeichnet, daß** man
a) eine Verbindung der Formel (II) 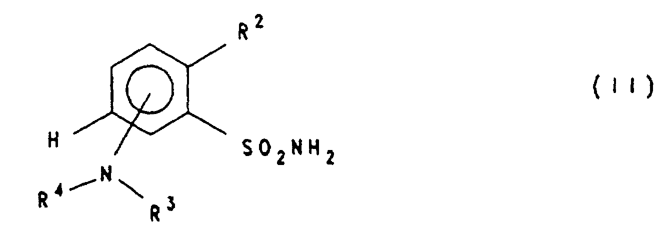 mit einem heterocyclischen Carbamat der Formel (III), 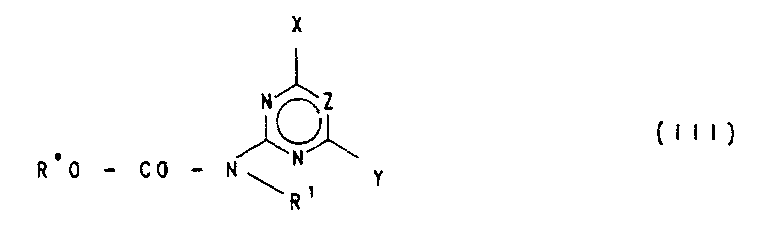 worin R* gegebenenfalls substituiertes Phenyl oder C₁-C₄-Alkyl bedeutet, umsetzt oder
b) ein Sulfonylisocyanat der Formel (IV) 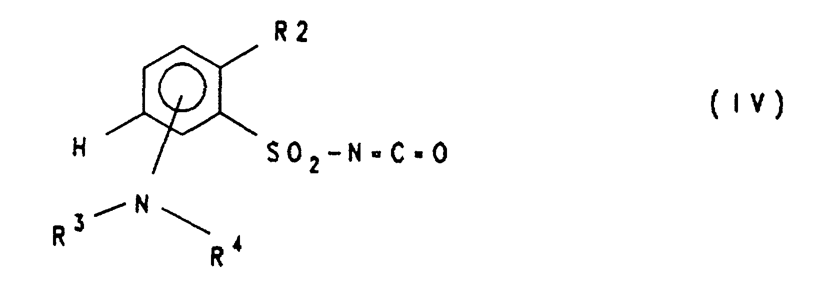 mit einem Aminoheterocyclus der Formel (V) 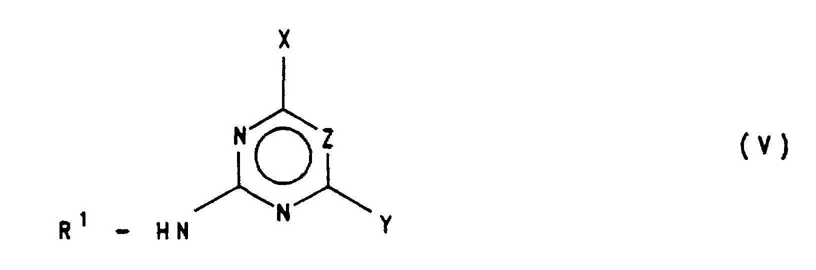 umsetzt, oder
c) ein Sulfochlorid der Formel (VI) 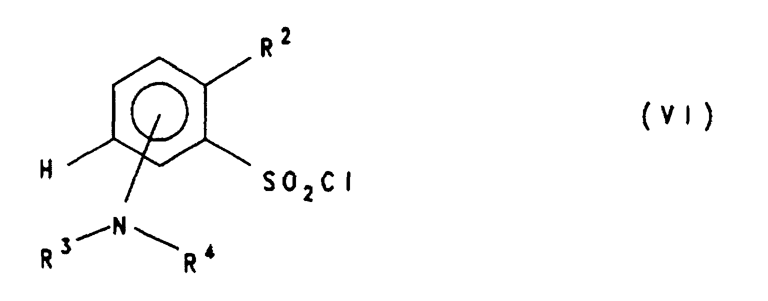 mit einem Aminoheterocyclus der genannten Formel (V) in Gegenwart eines Isocyanat-Salzes umsetzt oder
d) ein Sulfonamid der genannten Formel (II) mit einem (Thio-}Isocyanat der Formel (XIX) 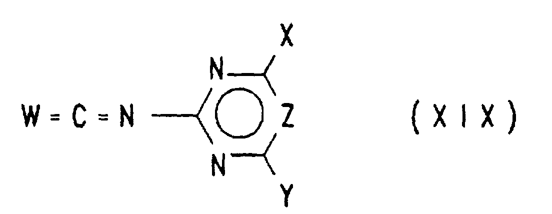 in Gegenwart einer Base umsetzt,
wobei in den obigen Formeln (II) bis (VI) und (XIX) die R¹, R², W, X, Y und Z wie in Formel (I) definiert sind und, im Falle von Verbindungen der Formel (I) mit G = (G1), R³ und R⁴ die Bedeutung und Position wie R^{3a} bzw. R^{4a} in Formel (G1) haben und, im Falle von Verbindungen der Formel (I) mit G = (G3), R³ und R⁴ die Bedeutung und Position wie R^{3b} bzw. R^{4b} in Formel (G3) haben.

6. Verbindungen der Formel (II) wie sie in Anspruch 5 definiert sind.

7. Verbindungen der Formel (IV) wie sie in Anspruch 5 definiert sind.

8. Verbindungen der Formel (VI) wie sie in Anspruch 5 definiert sind.

9. Herbizide oder pflanzenwachstumsregulierende Mittel, **dadurch gekennzeichnet, daß** sie mindestens eine Verbindung der Formel (I) oder deren Salz nach einem der Ansprüche 1 bis 4 und übliche Formulierungshilfsmittel enthalten.

10. Verwendung der Verbindungen der Formel (I) nach einem der Ansprüche 1 bis 4 als Herbizide oder Pflanzenwachstumsregulatoren.

11. Verfahren zur Bekämpfung von Schadpflanzen oder zur Wachstumsregulierung von Pflanzen, **dadurch gekennzeichnet, daß** man eine oder mehrere Verbindungen der Formel (I) nach einem der Ansprüche 1 bis 4 auf die Pflanzen, Pflanzensamen oder den Erdboden, auf oder in dem die Pflanzen wachsen, appliziert.

## Claims

1. A compound of the formula (I) or a salt thereof 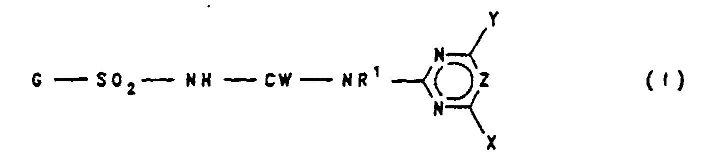 in which
G is a substituted N-acylaminophenyl radical from the group consisting of G¹ and G³ 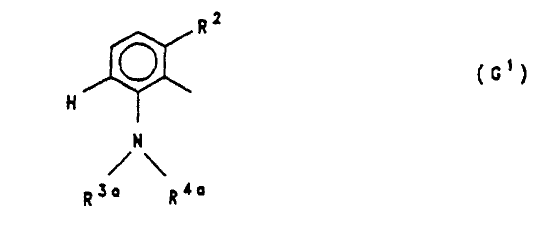 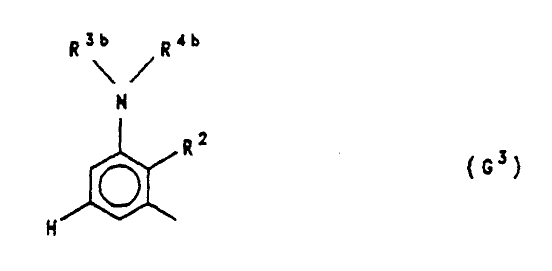
R¹ is H or C₁-C₃-alkyl,
R² is CO-OR⁵, CS-SR⁶, CO-SR⁷, CS-OR⁸, CO-R⁹, CO-NR¹⁰R¹¹, CO-O-N=CR¹²R¹³, C(=NR¹⁴)R¹⁵ or CS-NR¹⁶R¹⁷,
R^{3a} is C₁-C₅-alkyl, C₂-C₅-alkenyl or C₂-C₅-alkynyl, the three latter radicals being unsubstituted or substituted by one or more radicals from the group consisting of halogen, C₁-C₄-alkoxy, CN, di-(C₁-C₄-alkyl)amino, N₃, and C₁-C₃-alkylthio,
R^{3b} is C₁-C₅-alkyl, C₃-C₆-cycloalkyl, C₂-C₅-alkenyl or C₂-C₅-alkynyl, the four latter radicals being unsubstituted or substituted by one or more radicals from the group consisting of halogen, C₁-C₄-alkoxy, CN, di-(C₁-C₄-alkyl)amino, N₃ and C₁-C₃-alkylthio,
R^{4a} is CO-(C₁-C₅-alkyl) or CO₂-(C₁-C₅-alkyl), the two latter radicals being unsubstituted or substituted by one or more radicals from the group consisting of OH, NR¹⁸R¹⁹, N₃, CN, CO₂H, S(O)ₓ-(C₁-C₄-alkyl) and CO₂-(C₁-C₃-alkyl), or aminocarbonyl or aminosulfonyl, the two latter radicals being unsubstituted or N-substituted by identical or different radicals from the group consisting of C₁-C₄-alkyl and C₁-C₄-haloalkyl,
R^{4b} is CO-(C₁-C₅-alkyl), CO-(C₃-C₆-cycloalkyl), CO₂-(C₁-C₅-alkyl), CS- (C₁-C₅-alkyl), CO-S-(C₁-C₆-alkyl), CS-O-(C₁-C₆-alkyl) or CS-S-(C₁-C₆-alkyl), the seven latter radicals being unsubstituted or substituted by one or more radicals from the group consisting of CN, N₃, CO₂H, CO₂-(C₁-C₃-alkyl), OH, NR²⁰R²¹, S(O)ₓ-(C₁-C₄-alkyl) and CO-NR²²R²³, or SO₂NR²⁹R³⁰,
R⁵ is C₁-C₅-alkyl, C₂-C₅-alkenyl, C₂-C₅-alkynyl, C₃-C₆-cycloalkyl or C₄-C₇-cycloalkylalkyl, the latter five radicals being unsubstituted or substituted by one or more radicals from the group consisting of halogen, CN, N₃, C₁-C₃-alkoxy, C₁-C₃-alkylthio, amino, mono- and di-(C₁-C₄-alkyl)amino, NO₂, SCN, C₁-C₃-haloalkoxy and C₁-C₃-haloalkylthio,
R⁶ is a radical as for R⁵,
R⁷ is a radical as for R⁵,
R⁸ is a radical as for R⁵,
R⁹ is H, C₁-C₆-alkyl, C₂-C₆-alkenyl, C₂-C₆-alkynyl, C₃-C₇-cycloalkyl, the four latter radicals independently of one another being unsubstituted or substituted by one or more radicals from the group consisting of halogen, C₁-C₄-alkoxy, C₁-C₄-alkylthio, C₁-C₄-haloalkoxy, C₁-C₄-haloalkylthio and CN,
R¹⁰ is H, C₁-C₆-alkyl, C₂-C₆-alkenyl, C₂-C₆-alkynyl, the three latter radicals being unsubstituted or substituted by one or more radicals from the group consisting of C₁-C₃-alkoxy, C₁-C₃-alkylthio, halogen, CN, amino, C₁-C₄-alkylamino and di-(C₁-C₄-alkyl)amino, or C₁-C₄-alkoxy or OH,
R¹¹ is H, C₁-C₆-alkyl, C₂-C₆-alkenyl, C₂-C₆-alkynyl, the three latter radicals being unsubstituted or substituted by one or more radicals from the group consisting of halogen, CN, NH₂, mono-and di-(C₁-C₄-alkyl)amino, C₁-C₃-alkoxy and C₁-C₃-alkylthio, or
NR¹⁰R¹¹ together is a heterocyclic ring which in addition to the ring nitrogen atom may also contain up to two ring heteroatoms from the group consisting of N, O and S and may be further substituted,
R¹² and R¹³ independently of one another are H, C₁-C₄-alkyl, C₂-C₄-alkenyl or C₂-C₄-alkynyl, or
R¹² and R¹³ together are an alkylene chain of 3 or 4 carbon atoms, which may also be substituted by C₁-C₄-alkyl or C₁-C₄-alkoxy,
R¹⁴ is H, OH, NH₂, NHR³¹, NR³¹₂, C₁-C₄-alkyl, C₁-C₄-alkoxy, C₂-C₄-alkenyl or C₂-C₄-alkynyl, the four latter radicals being unsubstituted or substituted by one or more radicals from the group consisting of halogen, C₁-C₃-alkoxy and C₁-C₃-alkylthio,
R¹⁵ is H, C₁-C₆-alkyl, C₁-C₆-alkoxy, C₂-C₆-alkenyl, C₂-C₆-alkynyl, the four latter radicals being unsubstituted or substituted by one or more radicals from the group consisting of halogen, C₁-C₃-alkoxy and C₁-C₃-alkylthio,
R¹⁶ is a radical as for R¹⁰,
R¹⁷ is a radical as for R¹¹,
R¹⁸ independently of R¹⁹ is H, C₁-C₄-alkyl, C₂-C₅-alkenyl or C₂-C₅-alkynyl, the three latter radicals being unsubstituted or substituted by one or more radicals from the group consisting of halogen, C₁-C₃-alkoxy, C₁-C₃-alkylthio and CN,
R¹⁹ independently of R¹⁸ is a radical as for R¹⁸ or is C₁-C₃-alkoxy or OH, or
NR¹⁸R¹⁹ together is a heterocyclic ring which may in addition to the ring nitrogen atom contain up to two ring heteroatoms from the group consisting of N, O and S and may be further substituted,
R²⁰ independently of R²¹ is H, C₁-C₄-alkyl, C₂-C₄-alkenyl or C₂-C₄-alkynyl, the three latter radicals being unsubstituted or substituted by one or more radicals from the group consisting of halogen, CN, NH₂, mono- and di(C₁-C₄)-alkylamino, C₁-C₃-alkoxy, C₁-C₃-alkylthio and CO₂-(C₁-C₃-alkyl),
R²¹ independently of R²⁰ is a radical as for R²⁰ or is CHO, CO-(C₁-C₅-alkyl) or CO₂-(C₁-C₅-alkyl) or
NR²⁰R²¹ is a heterocyclic ring analogous to NR¹⁸R¹⁹,
R²² and R²³ independently of one another are H, C₁-C₃-alkyl, C₂-C₄-alkenyl or C₂-C₄-alkynyl or
NR²²R²³ is a heterocyclic ring analogous to NR¹⁸R¹⁹,
R²⁹ independently of R³⁰ is H, C₁-C₄-alkyl, C₂-C₅-alkenyl or C₂-C₅-alkynyl, the three latter radicals being unsubstituted or substituted by one or more radicals from the group consisting of halogen, C₁-C₃-alkoxy, C₁-C₃-alkylthio and CN,
R³⁰ independently of R²⁹ is H, C₁-C₄-alkyl, C₂-C₅-alkenyl or C₂-C₅-alkynyl, the three latter radicals being unsubstituted or substituted by one or more radicals from the group consisting of halogen, C₁-C₃-alkoxy, C₁-C₃-alkylthio and CN, or is C₁-C₃-alkoxy or OH, or
NR²⁹R³⁰ is a heterocyclic ring analogous to NR¹⁸R¹⁹,
R³¹ is C₁-C₄-alkyl, C₂-C₄-alkenyl or C₂-C₄-alkynyl, the three latter radicals independently of one another being unsubstituted or substituted by one or more radicals from the group consisting of halogen, C₁-C₄-alkoxy and C₁-C₄-alkylthio,
x - independently of the other indices x - is 0, 1 or 2,
W is O or S,
X and Y independently of one another are hydrogen, halogen, C₁-C₂-alkyl, C₁-C₂-alkoxy or C₁-C₂-alkylthio, the three latter radicals being unsubstituted or substituted by one or more radicals from the group consisting of halogen, C₁-C₂-alkoxy and C₁-C₂-alkylthio, or are mono- or di-(C₁-C₂-alkyl)amino, C₃-C₄-alkenyl, C₃-C₄-alkenyloxy or C₃-C₄-alkynyloxy, and
Z is CH or N.

2. A compound or salt thereof as claimed in claim 1, wherein
G is a radical of the formula (G1) and
R¹ is H or C₁-C₃-alkyl,
R² is CO-OR⁵, CS-SR⁶, CO-SR⁷, CS-OR⁸, CO-R⁹, CO-NR¹⁰R¹¹, CO-O-N=CR¹²R¹³, C(=NR¹⁴)R¹⁵ or CS-NR¹⁶R¹⁷
R^{3a} is C₁-C₄-alkyl, C₁-C₄-haloalkyl, C₂-C₄-alkenyl or C₂-C₄-alkynyl,
R^{4a} is CO-(C₁-C₄-alkyl), CO₂-(C₁-C₅-alkyl), CO-NH₂, CO-NH(C₁-C₄-alkyl), CO-N(C₁-C₄-alkyl)₂ or SO₂-NH₂, SO₂-NH(C₁-C₄-alkyl) or SO₂-N(C₁-C₄-alkyl)₂,
R⁵ is C₁-C₄-alkyl, C₁-C₄-haloalkyl, C₂-C₄-alkenyl, C₂-C₄-alkynyl, C₃-C₆-cycloalkyl or C₄-C₇-cycloalkylmethyl,
R⁶ is a radical as for R⁵
R⁷ is a radical as for R⁵,
R⁸ is a radical as for R⁵,
R⁹ is H, C₁-C₄-alkyl, C₂-C₄-alkenyl, C₂-C₄-alkynyl or C₃-C₆-cycloalkyl, the four latter radicals independently of one another being unsubstituted or substituted by one or more radicals from the group consisting of halogen, C₁-C₂-alkoxy, C₁-C₂-alkylthio, C₁-C₂-haloalkoxy, C₁-C₂-haloalkylthio and CN,
R¹⁰ and R¹¹ independently of one another are H, C₁-C₄-alkyl, C₂-C₄-alkenyl or C₂-C₄-alkynyl,
R¹² and R¹³ independently of one another are H, C₁-C₄-alkyl, C₂-C₄-alkenyl or C₂-C₄-alkynyl or
R¹² and R¹³ together are an alkylene chain of 3 or 4 carbon atoms, which may also be substituted by C₁-C₄-alkyl or C₁-C₄-alkoxy,
R¹⁴ is NH₂, NHR³¹, NR³¹₂, C₁-C₄-alkyl, C₁-C₄-alkoxy, C₂-C₄-alkenyl or C₂-C₄-alkynyl,
R¹⁵ is H, C₁-C₄-alkyl, C₁-C₄-alkoxy, C₂-C₄-alkenyl or C₂-C₄-alkynyl,
R¹⁶ is a radical as for R¹⁰,
R¹⁷ is a radical as for R¹¹,
R³¹ is C₁-C₄-alkyl, C₂-C₄-alkenyl or C₂-C₄-alkynyl,
W is O or S,
X and Y independently of one another are halogen, C₁-C₂-alkyl, C₁-C₂-haloalkyl, C₁-C₂-alkoxy, C₁-C₂-haloalkoxy, C₁-C₂-alkylthio, mono- or di-(C₁-C₂-alkyl)amino, C₃-C₄-alkenyl, C₃-C₄-alkenyloxy or C₃-C₄-alkynyloxy; and
Z is CH or N.

3. A compound or salt thereof as claimed in claim 1 or 2, wherein
G is a radical of the formula (G3), and
R¹ is H or C₁-C₃-alkyl,
R² is CO-OR⁵, CS-SR⁶, CO-SR⁷, CS-OR⁸, CO-R⁹, CO-NR¹⁰R¹¹, CO-O-N=CR¹²R¹³, C(=NR¹⁴)R¹⁵ or CS-NR¹⁶R¹⁷,
R^{3b} is C₁-C₄-alkyl, C₃-C₆-cycloalkyl, C₁-C₄-haloalkyl, C₂-C₄-alkenyl or C₂-C₄-alkynyl,
R^{4b} is CO-(C₁-C₄-alkyl), CO-(C₃-C₆-cycloalkyl) or CO₂-(C₁-C₄-alkyl), the 3 latter radicals being unsubstituted or substituted by one or more radicals from the group consisting of CN, NR²⁰R²¹, S(O)ₓ-(C₁-C₄-alkyl) and CO-NR²²R²³, or is SO₂NR²⁹R³⁰,
R⁵ is C₁-C₄-alkyl, C₂-C₄-alkenyl, C₂-C₄-alkynyl, C₃-C₆-cycloalkyl or C₄-C₇-cycloalkylmethyl, the five latter radicals being unsubstituted or substituted by one or more radicals from the group consisting of halogen, C₁-C₂-alkoxy and C₁-C₂-haloalkoxy,
R⁶ is a radical as for R⁵,
R⁷ is a radical as for R⁵,
R⁸ is a radical as for R⁵,
R⁹ is H, C₁-C₄-alkyl, C₂-C₄-alkenyl, C₂-C₄-alkynyl or C₃-C₆-cycloalkyl, the four latter radicals independently of one another being unsubstituted or substituted by one or more radicals from the group consisting of halogen, C₁-C₂-alkoxy, C₁-C₂-alkylthio, C₁-C₂-haloalkoxy, C₁-C₂-haloalkylthio and CN,
R¹⁰ and R¹¹ independently of one another are H, C₁-C₄-alkyl, C₂-C₄-alkenyl or C₂-C₄-alkynyl,
R¹² and R¹³ independently of one another are H, C₁-C₄-alkyl, C₂-C₄-alkenyl or C₂-C₄-alkynyl, or
R¹² and R¹³ together are an alkylene chain of 3 or 4 carbon atoms, which may also be substituted by C₁-C₄-alkyl or C₁-C₄-alkoxy,
R¹⁴ is NH₂, NHR³¹, NR³¹₂, C₁-C₄-alkyl, C₁-C₄-alkoxy, C₂-C₄-alkenyl or C₂-C₄-alkynyl,
R¹⁵ is H, C₁-C₄-alkyl, C₁-C₄-alkoxy, C₂-C₄-alkenyl or C₂-C₄-alkynyl,
R¹⁶ is a radical as for R¹⁰
R¹⁷ is a radical as for R¹¹,
R²⁰ independently of R²¹ is H, C₁-C₄-alkyl, C₂-C₄-alkenyl or C₂-C₄-alkynyl, the three latter radicals being unsubstituted or substituted by one or more radicals from the group consisting of halogen and C₁-C₂-alkoxy,
R²¹ independently of R²⁰ is a radical as for R²⁰ or is CHO, CO-(C₁-C₄-alkyl) or CO₂-(C₁-C₄-alkyl) or
NR²⁰R²¹ together is a heterocyclic ring which, in addition to the ring nitrogen atom, may also contain a ring heteroatom from the group consisting of N, O and S, and may be substituted further by radicals from the group consisting of C₁-C₄-alkyl, C₁-C₄-alkoxy and halogen, and may, where there is a ring heteroatom S, be oxidized at the S atom,
R²² and R²³ independently of one another are H, C₁-C₃-alkyl, C₂-C₄-alkenyl or C₂-C₄-alkynyl or
NR²²R²³ is a heterocyclic ring analogous to NR²⁰R²¹,
R²⁹ independently of R³⁰ is H, C₁-C₄-alkyl, C₂-C₄-alkenyl, C₂-C₄-alkynyl, the three latter radicals being unsubstituted or substituted by one or more radicals from the group consisting of halogen, C₁-C₃-alkoxy, C₁-C₃-alkylthio and CN,
R³⁰ independently of R²⁹ is H, C₁-C₄-alkyl, C₂-C₄-alkenyl or C₂-C₄-alkynyl, the three latter radicals being unsubstituted or substituted by one or more radicals from the group consisting of halogen, C₁-C₃-alkoxy, C₁-C₃-alkylthio and CN, or is C₁-C₃-alkoxy or OH, or
NR²⁹R³⁰ is a heterocyclic ring analogous to NR²⁰R²¹,
R³¹ is C₁-C₄-alkyl, C₂-C₄-alkenyl or C₂-C₄-alkynyl,
x is 0, 1 or 2,
W is O or S,
X and Y independently of one another are halogen, C₁-C₂-alkyl, C₁-C₂-haloalkyl, C₁-C₂-alkoxy, C₁-C₂-haloalkoxy, C₁-C₂-alkylthio, mono- or di-(C₁-C₂-alkyl)amino, C₃-C₄-alkenyl, C₃-C₄-alkenyloxy or C₃-C₄-alkynyloxy, and
Z is CH or N.

4. A compound or a salt thereof as claimed in any one of claims 1 to 3, wherein R² is a radical of the formula CO-OR⁵.

5. A process for the preparation of compounds of the formula (I) or salts thereof as defined in any one of claims 1 to 4, which comprises
a) reacting a compound of the formula (II) 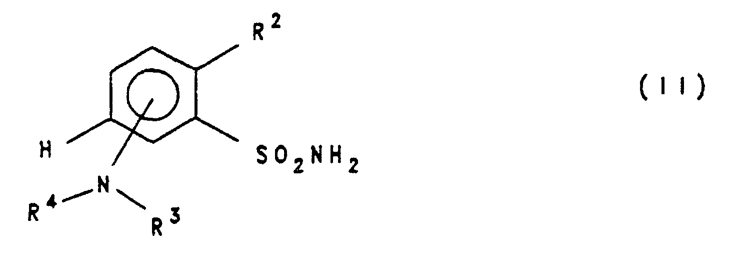 with a heterocyclic carbamate of the formula (III) 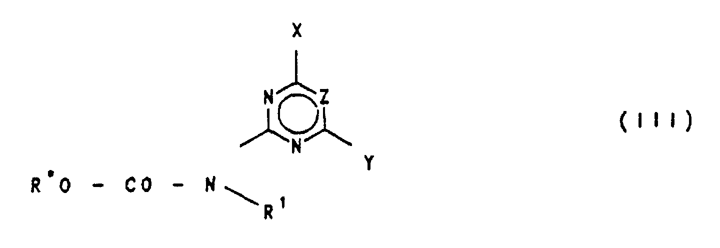 in which R* is unsubstituted or substituted phenyl or C₁-C₄-alkyl, or
b) reacting a sulfonyl isocyanate of the formula (IV) 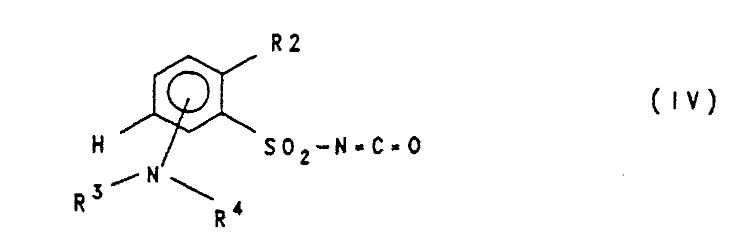 with an amino heterocycle of the formula (V) 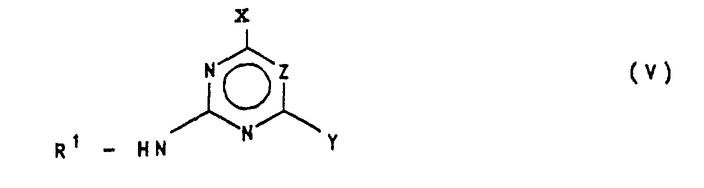 or
c) reacting a sulfonyl chloride of the formula (VI) 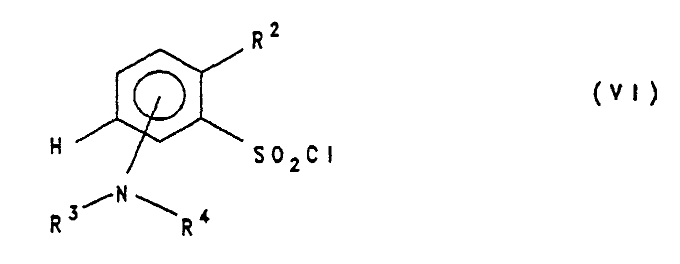 with an amino heterocycle of the abovementioned formula (V) in the presence of an isocyanate salt, or
d) reacting a sulfonamide of the abovementioned formula (II) with a (thio)isocyanate of the formula (XIX) 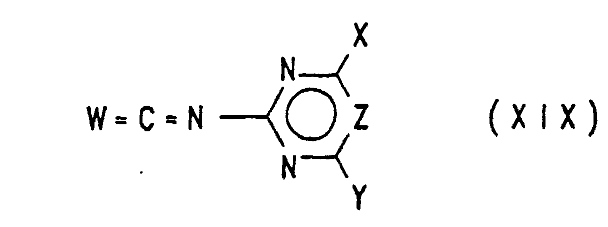 in the presence of a base,
the definitions of R¹, R², W, X, Y and Z in the above formulae (II) to (VI) and (XIX) being as for formula (I) and, in the case of compounds of the formula (I) where G = (G1), R³ and R⁴ having the definition and position as for R^{3a} and R^{4a} respectively in formula (G1), and, in the case of compounds of the formula (I) where G = (G3), R³ and R⁴ having the definition and position as for R^{3b} and R^{4b} respectively in formula (G3).

6. A compound of the formula (II) as defined in claim 5.

7. A compound of the formula (IV) as defined in claim 5.

8. A compound of the formula (VI) as defined in claim 5.

9. A herbicidal or plant growth-regulatory composition which contains at least one compound of the formula (I) or salt thereof as claimed in any one of claims 1 to 4 and conventional formulation auxiliaries.

10. The use of a compound of the formula (I) as claimed in any one of claims 1 to 4 as a herbicide or plant growth regulator.

11. A method of combating harmful plants or for controlling the growth of plants, which comprises applying one or more compounds of the formula (I) as claimed in any one of claims 1 to 4 to the plants, plant seeds or the soil on or in which the plants are growing.

## Revendications

1. Composés de formule (I) et leurs sels 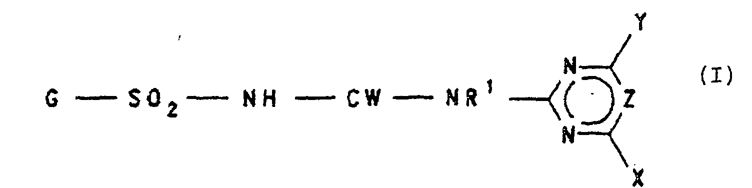 où
G représente un reste N-acylaminophényle substitué pris dans les groupes G¹ et G³, 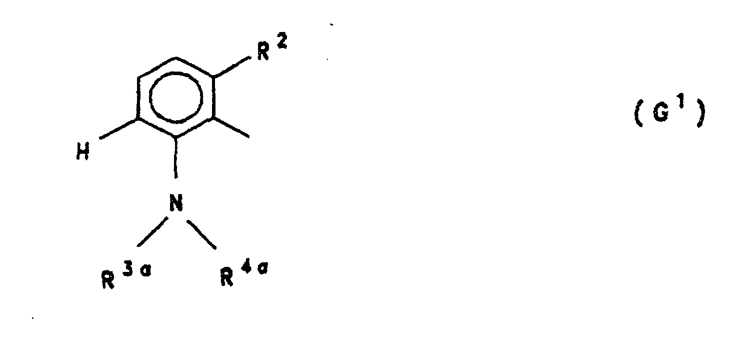 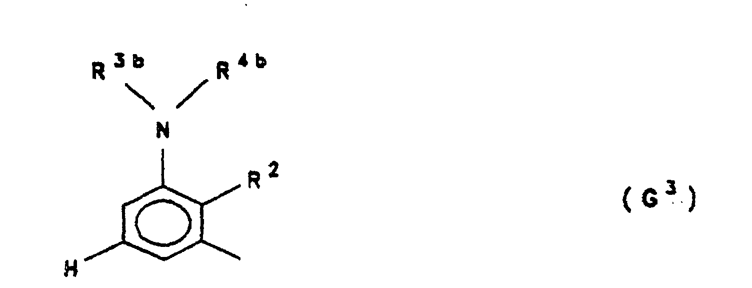
R¹ représente un atome d'hydrogène ou un groupe alkyle en C₁-C₃,
R² représente des groupes CO-OR⁵, CS-SR⁶, CO-SR⁷, CS-OR⁸, CO-R⁹, CO-NR¹⁰R¹¹, CO-O-N=CR¹²R¹³, C(=NR¹⁴)R¹⁵ ou CS-NR¹⁶R¹⁷,
R^{3a} représente des groupes alkyle en C₁-C₅, alcényle en C₂-C₅ ou alcynyle en C₂-C₅, les trois derniers restes cités étant non substitués ou substitués par un ou plusieurs restes pris dans le groupe des restes halogène, alkoxy en C₁-C₄, CN, di-(alkyl en C₁-C₄)amino, N₃ et (alkyl en C₁-C₃)thio,
R^{3b} alkyle en C₁-C₅, cycloalkyle en C₃-C₆, alcényle en C₂-C₅ ou alcynyle en C₂-C₅, les quatre derniers restes cités étant non substitués ou substitués par un ou plusieurs restes pris dans le groupe des restes halogène, alkoxy en C₁-C₄, CN, di-(alkyl en C₁-C₄)amino, N₃ et (alkyl en C₁-C₃)thio,
R^{4a} représente des groupes CO-(alkyle en C₁-C₅) ou CO₂-(alkyle en C₁-C₅),
les deux derniers restes cités étant non substitués ou substitués par un ou plusieurs restes pris dans le groupe des substituants OH, NR¹⁸R¹⁹, N₃, CN, CO₂H, S(O)ₓ-(alkyle en C₁-C₄) et CO₂-alkyle en C₁-C₃,
ou aminocarbonyle ou aminosulfonyle, les deux derniers restes cités étant non substitués ou N-substitués par des restes identiques ou différents pris dans le groupe des restes alkyle en C₁-C₄ et haloalkyle en C₁-C₄,
R^{4b} représente des groupes CO-(alkyle en C₁-C₅), CO-(cycloalkyle en C₃-C₆), CO₂-(alkyle en C₁-C₅), CS-(alkyle en C₁-C₅) CO-S-(alkyle en C₁-C₆), CS-O-(alkyle en C₁-C₆) ou CS-S-(alkyle en C₁-C₆), les sept derniers restes cités étant non substitués ou substitués par un ou plusieurs restes pris dans le groupe des restes CN, N₃, CO₂H, CO₂-(alkyle en C₁-C₃), OH, NR²⁰R²¹, S(O)x-(alkyle en C₁-C₄), CO-NR²²R²³
ou SO₂NR²⁹R³⁰,
R⁵ représente des groupes alkyle en C₁-C₅, alcényle en C₂-C₅, alcynyle en C₂-C₅, cycloalkyle en C₃-C₆ ou cycloalkylalkyle en C₄-C₇, les cinq derniers restes cités étant non substitués ou substitués par un ou plusieurs restes pris dans le groupe des restes halogène, CN, N₃, alkoxy en C₁-C₃, (alkyl en C₁-C₃)thio, amino, mono- et , di-(alkyl en C₁-C₄)-amino, NO₂, SCN, haloalkoxy en C₁-C₃ et halo(alkyl en C₁-C₃)thio,
R⁶ représente un reste comme R⁵,
R⁷ représente un reste comme R⁵,
R⁸ représente un reste comme R⁵,
R⁹ représente un atome d'hydrogène, des groupes alkyle en C₁-C₆, alcényle en C₂-C₆, alcynyle en C₂-C₆, cycloalkyle en C₃-C₆, les quatre derniers restes cités, indépendamment les uns des autres, étant non substitués ou substitués par un ou plusieurs restes pris dans le groupe des restes halogène, alkoxy en C₁-C₄, (alkyl en C₁-C₄)thio, haloalkoxy en C₁-C₄, halo(alkyl en C₁-C₄)thio et CN,
R¹⁰ représente un atome d'hydrogène, des groupes alkyle en C₁-C₆, alcényle en C₂-C₆, alcynyle en C₂-C₆, les trois derniers restes cités étant non substitués ou substitués par un ou plusieurs restes pris dans le groupe des restes alkoxy en C₁-C₃, (alkyl en C₁-C₃)-thio, halogène, CN, amino, (alkyl en C₁-C₄)amino et di-(alkyl en C₁-C₄)amino, ou représente alkoxy en C₁-C₄ ou OH,
R¹¹ représente un atome d'hydrogène, des groupes alkyle en C₁-C₆, alcényle en C₂-C₆, alcynyle en C₂-C₆, les trois derniers restes cités étant non substitués ou substitués par un ou plusieurs restes pris dans le groupe des restes halogène, CN, NH₂, mono- ou di-(alkyl en C₁-C₄)amino, alkoxy en C₁-C₃ et (alkyl en C₁-C₃)thio ou
NR¹⁰R¹¹ forment ensemble un cycle hétérocyclique, qui peut contenir encore, au côté de l'atome nucléaire N, jusqu'à deux hétéroatomes nucléaires pris parmi les atomes de N, de O et de S et peut être substitué davantage,
R¹² et R¹³ représentent, indépendamment l'un de l'autre, un atome d'hydrogène, des groupes alkyle en C₁-C₄, alcényle en C₂-C₄ ou alcynyle en C₂-C₄ ou
R¹² et R¹³ représentent ensemble une chaîne alkylène de 3 ou 4 atomes de carbone, qui peut être substituée encore par des substiuants alkyle en C₁-C₄ ou alkoxy en C₁-C₄,
R¹⁴ représente un atome d'hydrogène, des groupes OH, NH₂, NHR³¹, NR³¹₂, alkyle en C₁-C₄, alkoxy en C₁-C₄, alcényle en C₂-C₄ ou alcynyle en C₂-C₄, les quatre derniers restes cités étant non substitués ou substitués par un ou plusieurs restes pris dans le groupes des restes halogène, alkoxy en C₁-C₃ et (alkyl en C₁-C₃)thio,
R¹⁵ représente un atome d'hydrogène, alkyle en C₁-C₆, alkoxy en C₁-C₆, alcényle en C₂-C₆, alcynyle en C₂-C₆, les quatre derniers restes cités étant non substitués ou substitués par un ou plusieurs restes pris parmi les restes halogène, alkoxy en C₁-C₃, (alkyl en C₁-C₃)thio,
R¹⁶ représente un reste comme R¹⁰,
R¹⁷ représente un reste comme R¹¹,
R¹⁸ représente, indépendamment de R¹⁹, un atome d'hydrogène, des groupes alkyle en C₁-C₄, alcényle en C₂-C₅ ou alcynyle en C₂-C₅, les trois derniers restes cités étant non substitués ou substitués par un ou plusieurs restes pris parmi les restes halogène, alkoxy en C₁-C₃, (alkyl en C₁-C₃)thio et CN,
R¹⁹ représente, indépendamment de R¹⁸, un reste comme R¹⁸ ou alkoxy en C₁-C₃ ou OH, ou
NR¹⁸R¹⁹ représentent ensemble un cycle hétérocyclique qui peut contenir encore, au côté de l'atome nucléaire N, jusqu'à deux hétéroatomes nucléaires pris parmi les atomes de N, de O ou de S et peut être substitué davantage,
R²⁰ représente, indépendamment de R²¹, un atome d'hydrogène, des groupes alkyle en C₁-C₄, alcényle en C₂-C₄ ou alcynyle en C₂-C₄, les trois derniers restes cités étant non substitués ou substitués par un ou plusieurs restes pris dans le groupe des restes halogène, CN, NH₂, mono- ou di-(alkyl en C₁-C₄)amino, alkoxy en C₁-C₃ ou (alkyl en C₁-C₃)thio ou CO₂-(alkyle en C₁-C₃),
R²¹ représente, indépendamment de R²⁰, un reste comme R²⁰ ou CHO, CO-(alkyle en C₁-C₅) ou CO₂-(alkyle en C₁-C₅) ou
NR²⁰R²¹ représente un cycle hétérocyclique analogue à NR¹⁸R¹⁹,
R²² et R²³ représentent, indépendamment l'un de l'autre, un atome d'hydrogène, des groupes alkyle en C₁-C₃, alcényle en C₂-C₄ ou alcynyle en C₂-C₄ ou
NR²²R²³ représente un cycle hétérocyclique analogue à NR¹⁸R¹⁹,
R²⁹ représente indépendamment de R³⁰, un atome d'hydrogène, des groupes alkyle en C₁-C₄, alcényle en C₂-C₅ ou alcynyle en C₂-C₅, les trois derniers restes cités étant non substitués ou substitués par un ou plusieurs restes pris dans le groupe des restes halogène, alkoxy en C₁-C₃, (alkyl en C₁-C₃)thio et CN,
R³⁰ représente, indépendamment de R²⁹, un atome d'hydrogène, des groupes alkyle en C₁-C₄, alcényle en C₂-C₅ ou alcynyle en C₂-C₅, les trois derniers restes cités étant non substitués ou substitués par un ou plusieurs restes pris dans le groupe des restes halogène, alkoxy en C₁-C₃, (alkyl en C₁-C₃)thio et CN, ou représente des groupes alkoxy en C₁-C₃ ou OH,
NR²⁹R³⁰ représente un cycle hétérocyclique analogue à NR¹⁸R¹⁹,
R³¹ représente des groupes alkyle en C₁-C₄, alcényle en C₂-C₄ ou alcynyle en C₂-C₄, les trois derniers restes cités étant, indépendammenet l'un de l'autre, non substitués ou substitués par un ou plusieurs restes pris dans le groupe des restes halogène, alkoxy en C₁-C₄ et (alkyl en C₁-C₄)thio,
x vaut - indépendamment des autres indices x - 0, 1 ou 2,
W représente des atomes de O ou de S,
X, Y représentent, indépendamment l'un de l'autre, un atome d'hydrogène, un atome d'halogène, des restes alkyle en C₁-C₂, alkoxy en C₁-C₂ ou (alkyl en C₁-C₂)-thio, les trois derniers restes cités étant non substitués ou substitués par un ou plusieurs restes pris dans le groupe des restes halogène, alkoxy en C₁-C₂ et (alkyl en C₁-C₂)thio, ou mono- ou di-(alkyl en C₁-C₂)amino, alcényle en C₃-C₄, (alcényl en C₃-C₄)oxy ou (alcynyl en C₃-C₄)oxy et
Z représente des groupes CH ou N.

2. Composés et leurs sels selon la revendication 1, **caractérisés en ce que**
G représente un reste de formule (G1) et
R¹ représente un atome d'hydrogène ou un groupe alkyle en C₁-C₃,
R² représente des groupes CO-OR⁵, CS-SR⁶, CO-SR⁷, CS-OR⁸, CO-R⁹, CO-NR¹⁰R¹¹, CO-O-N=CR¹²R¹³, C(=NR¹⁴)R¹⁵ ou CS-NR¹⁶R¹⁷,
R^{3a} représente des groupes alkyle en C₁-C₄, haloalkyle en C₁-C₄, alcényle en C₂-C₄ ou alcynyle en C₂-C₄,
R^{4a} représente des groupes CO-(alkyle en C₁-C₄), CO₂-(alkyle en C₁-C₅), CO-NH₂, CO-NH-(alkyle en C₁-C₄), CO-N(alkyle en C₁-C₄)₂ ou SO₂NH₂, SO₂-NH-(alkyle en C₁-C₄) ou SO₂-N(alkyle en C₁-C₄)₂,
R⁵ représente des groupes alkyle en C₁-C₄, haloalkyle en C₁-C₄, alcényle en C₂-C₄, alcynyle en C₂-C₄, cycloalkyle en C₃-C₆ ou cyclo(alkyl en C₄-C₇)-méthyle,
R⁶ représente un reste comme R⁵,
R⁷ représente un reste comme R⁵,
R⁸ représente un reste comme R⁵,
R⁹ représente un atome d'hydrogène, des groupes alkyle en C₁-C₄, alcényle en C₂-C₄, alcynyle en C₂-C₄ ou cycloalkyle en C₃-C₆, les quatre derniers restes cités, indépendamment les uns des autres, étant non substitués ou substitués par un ou plusieurs restes pris dans le groupe des restes halogène, alkoxy en C₁-C₂, (alkyl en C₁-C₂)thio, haloalkoxy en C₁-C₂ et halo(alkyl en C₁-C₂)thio et CN,
R¹⁰ et R¹¹ représentent, indépendamment l'un de l'autre, un atome d'hydrogène, des groupes alkyle en C₁-C₄, alcényle en C₂-C₄ ou alcynyle en C₂-C₄,
R¹² et R¹³ représentent, indépendamment l'un de l'autre, un atome d'hydrogène, des groupes alkyle en C₁-C₄, alcényle en C₂-C₄ ou alcynyle en C₂-C₄ ou
R¹² et R¹³ représentent ensemble une chaîne alkylène de 3 ou 4 atomes de carbone, qui peut être substituée encore par des substituants alkyle en C₁-C₄ ou alkoxy en C₁-C₄,
R¹⁴ représente des groupes NH₂, NHR³¹, NR³¹₂, alkyle en C₁-C₄, alkoxy en C₁-C₄, alcényle en C₂-C₄ ou alcynyle en C₂-C₄,
R¹⁵ représente un atome d'hydrogène, alkyle en C₁-C₄, alkoxy en C₁-C₄, alcényle en C₂-C₄ ou alcynyle en C₂-C₄,
R¹⁶ représente un reste comme R¹⁰,
R¹⁷ représente un reste comme R¹¹,
R³¹ représente des groupes alkyle en C₁-C₄, alcényle en C₂-C₄ ou alcynyle en C₂-C₄,
W représente des atomes de O ou de S,
X, Y représentent, indépendamment l'un de l'autre, un atome d'halogène, des restes alkyle en C₁-C₂, haloalkyle en C₁-C₂, alkoxy en C₁-C₂, haloalkoxy en C₁-C₂ ou (alkyl en C₁-C₂)thio, mono- ou di-(alkyl en C₁-C₂)amino, alcényle en C₃-C₄, (alcényl en C₃-C₄)-oxy ou (alcynyl en C₃-C₄)oxy, et
Z représente des groupes CH ou N.

3. Composés et leurs sels selon la revendication 1 ou 2, **caractérisés en ce que**
G représente un reste de formule (G3) et
R¹ représente un atome d'hydrogène ou un groupe alkyle en C₁-C₃,
R² représente des groupes CO-OR⁵, CS-SR⁶, CO-SR⁷, CS-OR⁸, CO-R⁹, CO-NR¹⁰R¹¹, CO-O-N=CR¹²R¹³, C(=NR¹⁴)R¹⁵ ou CS-NR¹⁶R¹⁷,
R^{3b} représente des groupes alkyle en C₁-C₄, cycloalkyle en C₃-C₆, haloalkyle en C₁-C₄, alcényle en C₂-C₄ ou alcynyle en C₂-C₄,
R^{4b} représente des groupes CO-(alkyle en C₁-C₄), CO-(cycloalkyle en C₃-C₆) ou CO₂-(alkyle en C₁-C₄), les trois derniers restes cités étant non substitués ou substitués par un ou plusieurs restes pris dans le groupe des restes CN, NR²⁰R²¹, S(O)ₓ-alkyle en C₁-C₄ et CO-NR²²R²³, ou SO₂NR²⁹R³⁰,
R⁵ représente des groupes alkyle en C₁-C₄, alcényle en C₂-C₄, alcynyle en C₂-C₄, cycloalkyle en C₃-C₆ ou cyclo(alkyl en C₄-C₇)-méthyle, les cinq derniers restes cités étant non substitués ou substitués par un ou plusieurs restes pris dans le groupe des reste halogène, alkoxy en C₁-C₂ et haloalkoxy en C₁-C₂,
R⁶ représente un reste comme R⁵,
R⁷ représente un reste comme R⁵,
R⁸ représente un reste comme R⁵,
R⁹ représente un atome d'hydrogène, des groupes alkyle en C₁-C₄, alcényle en C₂-C₄, alcynyle en C₂-C₄ ou cycloalkyle en C₃-C₆, les quatre derniers restes cités, indépendamment les uns des autres, étant non substitués ou substitués par un ou plusieurs restes pris dans le groupe des restes halogène, alkoxy en C₁-C₂, (alkyl en C₁-C₂)thio, haloalkoxy en C₁-C₂, halo(alkyl en C₁-C₂)thio et CN,
R¹⁰ et R¹¹ représentent, indépendamment l'un de l'autre, un atome d'hydrogène, des groupes alkyle en C₁-C₄, alcényle en C₂-C₄ ou alcynyle en C₂-C₄,
R¹² et R¹³ représentent, indépendamment l'un de l'autre, un atome d'hydrogène, des groupes alkyle en C₁-C₄, alcényle en C₂-C₄ ou alcynyle en C₂-C₄ ou
R¹² et R¹³ représentent ensemble une chaîne alkylène de 3 ou 4 atomes de carbone, qui peut être substituée encore par des substituants alkyle en C₁-C₄ ou alkoxy en C₁-C₄,
R¹⁴ représente des groupes NH₂, NHR³¹, NR³¹₂, alkyle en C₁-C₄, alkoxy en C₁-C₄, alcényle en C₂-C₄ ou alcynyle en C₂-C₄,
R¹⁵ représente un atome d'hydrogène, des restes alkyle en C₁-C₄, alkoxy en C₁-C₄, alcényle en C₂-C₄ ou alcynyle en C₂-C₄,
R¹⁶ représente un reste comme R¹⁰,
R¹⁷ représente un reste comme R¹¹,
R²⁰ représente, indépendamment de R²¹, un atome d'hydrogène, des groupes alkyle en C₁-C₄, alcényle en C₂-C₄ ou alcynyle en C₂-C₄, les trois derniers restes cités étant non substitués ou substitués par un ou plusieurs substituants pris dans le groupe des substituants halogène et alkoxy en C₁-C₂,
R²¹ représente, indépendamment de R²⁰, un reste comme R²⁰ ou des groupes CHO, CO-(alkyle en C₁-C₄) ou CO₂-(alkyle en C₁-C₄) ou
NR²⁰R²¹ représentent ensemble un cycle hétérocyclique, qui peut contenir encore, outre l'atome de N nucléaire, un hétéroatome nucléaire pris dans le groupes des atomes de N, de O et de S et peut être substitué encore par des restes pris dans le groupe des restes alkyle en C₁-C₄, alkoxy en C₁-C₄ et halogène et, dans le cas où S est un hétéroatome nucléaire, peut être oxydé sur l'atome de S,
R²² et R²³ représentent, indépendamment l'un de l'autre, un atome d'hydrogène, des groupes alkyle en C₁-C₃, alcényle en C₂-C₄ ou alcynyle en C₂-C₄ ou
NR²²R²³ représente un cycle hétérocyclique analogue à NR²⁰R²¹,
R²⁹ représente, indépendamment de R³⁰, un atome d'hydrogène, des groupes alkyle en C₁-C₄, alcényle en C₂-C₄, alcynyle en C₂-C₄, les trois derniers restes cités étant non substitués ou substitués par un ou plusieurs restes pris dans le groupe des restes halogène, alkoxy en C₁-C₃ ou (alkyl en C₁-C₃)-thio et CN,
R³⁰ représente, indépendamment de R²⁹, un atome d'hydrogène, des groupes alkyle en C₁-C₄, alcényle en C₂-C₄, alcynyle en C₂-C₄, les trois derniers restes cités étant non substitués ou substitués par un ou plusieurs restes pris dans le groupe des restes halogène, alkoxy en C₁-C₃, (alkyl en C₁-C₃)-thio et CN, ou alkoxy en C₁-C₃ ou OH ou
NR²⁹R³⁰ représente un cycle hétérocyclique analogue à NR²⁰R²¹,
R³¹ représente des groupes alkyle en C₁-C₄, alcényle en C₂-C₄ ou alcynyle en C₂-C₄,
x vaut 0, 1 ou 2,
W représente un atome de O ou de S,
X, Y représentent, indépendamment l'un de l'autre, un atome d'halogène, des restes alkyle en C₁-C₂, haloalkyle en C₁-C₂, alkoxy en C₁-C₂, haloalkoxy en C₁-C₂, (alkyl en C₁-C₂)thio, mono- ou di-(alkyl en C₁-C₂)amino, alcényle en C₃-C₄, (alcényl en C₃-C₄)-oxy ou (alcynyl en C₃-C₄)oxy, et
Z représente des groupes CH ou N.

4. Composés et leurs sels selon l'une des revendications 1 à 3, **caractérisés en ce que** R² représente un reste de formule CO-OR⁵.

5. Procédé pour la préparation de composés de formule (I) ou leurs sels, définis selon l'une des revendications 1 à 4, **caractérisé en ce qu'**on fait réagir
a) un composé de formule (II) 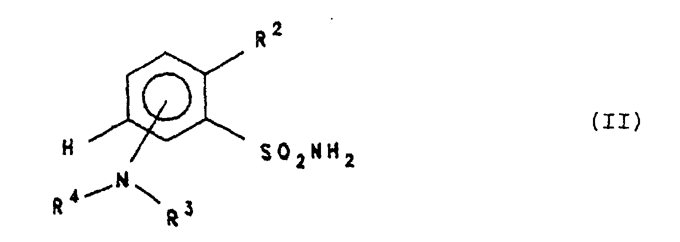 avec un carbamate hétérocyclique de formule (III), 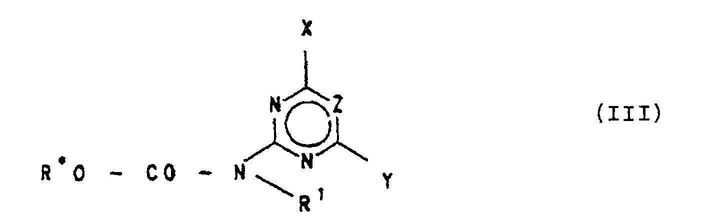 où R* représente un groupe alkyle en C₁-C₄ ou phényle éventuellement substitué ou
on fait réagir
b) un sulfonylisocyanate de formule (IV) 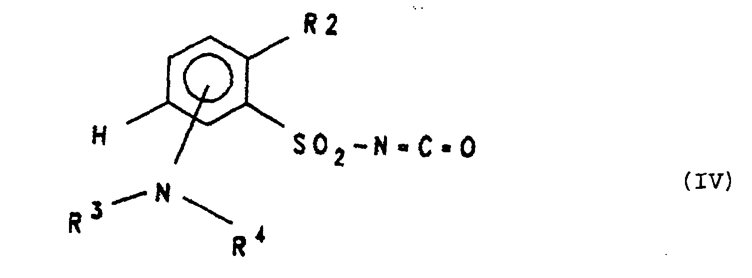 avec un hétérocycle aminé de formule (V) 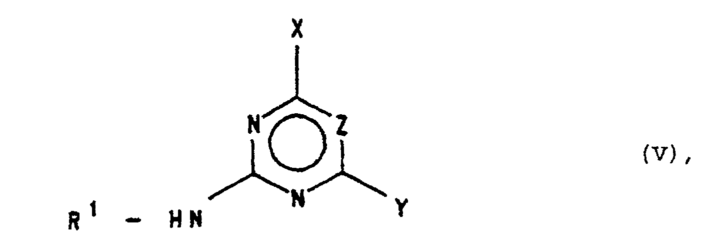 ou on fait réagir
c) un sulfochlorure de formule (VI) 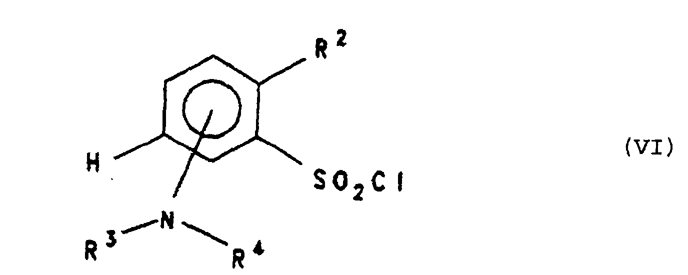 avec un hétérocycle aminé de formule (V) précitée en présence d'un sel d'isocyanate, par exemple d'un sel de Na ou de K, ou on fait réagir
d) un sulfonamide de formule (II) citée avec un (thio)isocyanate de formule (XIX) 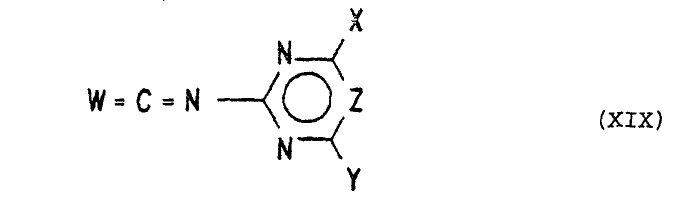 en présence d'une base, par exemple, le carbonate de potassium ou la triéthylamine,
dans les formules (II) à (VI) et (XIX) ci-dessus R¹, R², W, X, Y et Z sont définis comme à la formule (I) et, dans le cas de composés de formule (I) avec G = (G1), R³ et R⁴ possèdent la signification et sont en position comme R^{3a}
ou R^{4a} à la formule (G1), et dans le cas de composés de formule (I) avec G = (G3), R³ et R⁴ possèdent la signification et sont en position comme R^{3b} ou R^{4b} à la formule (G3).

6. Composés de formule (II) définis comme à la revendication 5.

7. Composés de formule (IV) définis comme à la revendication 5.

8. Composés de formule (VI) définis comme à la revendication 5.

9. Herbicides ou substances de croissance, **caractérisés en ce qu'**ils contiennent au moins un composé de formule (I) ou leur sel selon l'une des revendications 1 à 4 et des adjuvants de formulation usuels.

10. Utilisation des composés de formule (I) selon l'une des revendications 1 à 4 comme herbicides ou substances de croissance.

11. Procédé pour la lutte contre les mauvaises herbes ou pour la régulation de la croissance de plantes, **caractérisé en ce qu'**on applique un ou plusieurs composés de formule (I) selon l'une des revendications 1 à 4, sur les plantes, les grains de plantes ou le sol cultivable où poussent les plantes.
